# EUROPEAN PATENT SPECIFICATION

(11) **EP 1 999 268 B1**
(45) Date of publication and mention of the grant of the patent: **09.11.2016**
(21) Application number: 06815870.8
(22) Date of filing: 28.09.2006
(51) Int. Cl.: C12P 19/34, C12Q 1/68

(54) **NON-INVASIVE MOLECULAR COLONY METHODS, KITS AND APPARATUS**
NICHTINVASIVE MOLEKULARE KOLONIEVERFAHREN, KITS UND VORRICHTUNGEN
PROCÉDÉS, KITS ET APPAREILS NON INVASIFS POUR PRODUIRE DES COLONIES MOLÉCULAIRES

(30) Priority: 24.03.2006 RU 2006109271
(43) Date of publication of application: 10.12.2008
(73) Proprietor: Hone, William J., Irvington, NY 10533 (US); Chetverin, Alexander Borisovich, Puschino, Moscow Region 142290 (RU); Chetverina, Helena Vladimirovna, Pushchino, Moscow Region 142290 (RU); Samatov, Timur Rustemovich, Ufa 450015 (RU)
(72) Inventor: CHETVERIN, Alexander, Borisovich, Puschino, Moscow Region 142290 (RU); CHETVERINA, Helena, Vladimirovna, Pushchino, Moscow Region 142290 (RU); SAMATOV, Timur, Rustemovich, Ufa 450015 (RU)
(74) Representative: Chapman, Paul William
(86) International application number: PCT/US2006/038191
(87) International publication number: WO 2007/111639

(56) References cited:
- WO-A-03/000839
- US-A- 6 001 568
- US-A1- 2004 197 793
- SAMATOV T R ET AL: "Real-time monitoring of DNA colonies growing in a polyacrylamide gel" ANALYTICAL BIOCHEMISTRY, ACADEMIC PRESS INC, NEW YORK, vol. 356, no. 2, 15 September 2006 (2006-09-15), pages 300-302, XP024942046 ISSN: 0003-2697 [retrieved on 2006-09-15]
- CHETVERINA H V ET AL: "Nanocolonies: Detection, Cloning, and Analysis of Individual Molecules" BIOCHEMISTRY (MOSCOW), vol. 73, no. 13, December 2008 (2008-12), pages 1361-1387, XP002561569 ISSN: 0006-2979
- CHETVERINA H V ET AL: "Simultaneous assay of DNA and RNA targets in the whole blood using novel isolation procedure and molecular colony amplification" ANALYTICAL BIOCHEMISTRY, ACADEMIC PRESS INC, NEW YORK, vol. 334, no. 2, 15 November 2004 (2004-11-15), pages 376-381, XP004607620 ISSN: 0003-2697
- MITRA ROBI D ET AL: "Digital genotyping and haplotyping with polymerase colonies." PROCEEDINGS OF THE NATIONAL ACADEMY OF SCIENCES OF THE UNITED STATES OF AMERICA 13 MAY 2003, vol. 100, no. 10, 13 May 2003 (2003-05-13), pages 5926-5931, XP002561570 ISSN: 0027-8424

### LIST OF SEQUENCES

<110> Chetverin, Alexander Borisovich
   Samatov, Timur Rustemovich
   Chetverina, Helena Vladimirovna
   Hone, William J.
<120> Non-invasive molecular colony methods, kits and apparatus
<160> 14
<210> 1
   <211> 23
   <212> DNA
   <213> artificial sequence
<220> linear
   <223> forward primer YgfpF
<400> 1
   ggatccgttc aactagcaga cca 23
<210> 2
   <211> 25
   <212> DNA
   <213> artificial sequence
<220> linear
   <223> reverse primer YgfpR
<400> 2
   aatgatgatg atgatgatga gaacc 25
<210> 3
   <211> 229
   <212> DNA
   <213> artificial sequence
<220> linear
   <223> region of a plasmid carrying a sequence encoding the Cycle 3 mutant of green fluorescent protein
<400> 3
   ggatccgttc aactagcaga ccattatcaa caaaatactc caattggcga tggccctgtc  60
   cttttaccag acaaccatta cctgtcgaca caatctgccc tttcgaaaga tcccaacgaa 120
   aagagagacc acatggtcct tcttgagttt gtaacagctg ctgggattac acatggcatg 180
   gatgagctct acaaacccgg ggggggttct catcatcatc atcatcatt             229
<210> 4
   <211> 27
   <212> DNA
   <213> artificial sequence
<220> linear
   <223> probe Ydonor
<400> 4
   gggcagattg tgtcgacagg taatggt 27
<210> 5
   <211> 27
   <212> DNA
   <213> artificial sequence
<220> linear
   <223> probe Yacceptor
<400> 5
   tctggtaaaa ggacagggcc atcgcca 27
<210> 6
   <211> 27
   <212> DNA
   <213> artificial sequence
<220> linear
   <223> molecular beacon Y
<400> 6
   ccgcgtccat gccatgtgta atcccagcag cgcgg 35
<210> 7
   <211> 21
   <212> DNA
   <213> artificial sequence
<220> linear
   <223> primer AML1-A
<400> 7
   ctaccgcagc catgaagaac c 21
<210> 8
   <211> 21
   <212> DNA
   <213> artificial sequence
<220> linear
   <223> primer ETO-B
<400> 8
   agaggaaggc ccattgctga a 21
<210> 9
   <211> 395
   <212> DNA
   <213> artificial sequence
<220> linear
   <223> fragment of cDNA of chimeric AML1-ETO mRNA
<400> 9
   ctaccgcagc catgaagaac caggttgcaa gatttaatga cctcaggttt gtcggtcgaa  60
   gtggaagagg gaaaagcttc actctgacca tcactgtctt cacaaaccca ccgcaagtcg 120
   ccacctacca cagagccatc aaaatcacag tggatgggcc ccgagaacct cgaaaccgta 180
   ctgagaagca ctccacaatg ccagactcac ctgtggatgt gaagacgcaa tctaggctga 240
   ctcctccaac aatgccacct cccccaacta ctcaaggagc tccaagaacc agttcattta 300
   caccgacaac gttaactaat agcacgagcc attctcctac agtcttgaat ggcgccccct 360
   caccacccaa tggcttcagc aatgggcctt cctct                            395
<210> 10
   <211> 22
   <212> DNA
   <213> artificial sequence
<220> linear
   <223> primer AML1-C
<400> 10
   atgacctcag gtttgtcggt cg 22
<210> 11
   <211> 22
   <212> DNA
   <213> artificial sequence
<220> linear
   <223> primer ETO-D
<400> 11
   tgaactggtt cttggagctc ct 22
<210> 12
   <211> 27
   <212> DNA
   <213> artificial sequence
<220> linear
   <223> probe Ldonor
<400> 12
   tggcattgtg gagtgcttct cagtacg 27
<210> 13
   <211> 27
   <212> DNA
   <213> artificial sequence
<220> linear
   <223> probe Lacceptor
<400> 13
   ttcgaggttc tcggggccca tc 22
<210> 14
   <211> 25
   <212> DNA
   <213> artificial sequence
<220> linear
   <223> primer YF414
<400> 14
   tcaagtttga aggtgatacc cttgt 25

## Description

### TECHNICAL FIELD

This invention relates to primer-dependent amplification of nucleic acid molecules in immobilized media, and more particularly to detecting products of such amplification.

### BACKGROUND

Methods for in vitro exponential amplification of nucleic acids are well known. Commonly utilized methods are primer-dependent. The predominant primer-dependent method is the PCR, the polymerase chain reaction (U.S. patents 4,683,195, 4,683,202, 4,965,188). Amplification by PCR may be combined with reverse transcription, if the starting template is RNA (U.S. patent 5,310,652). Other known primer-dependent amplification methods include NASBA, nucleic acid sequence-based amplification (Compton, 1991), strand displacement amplification (Walker et al., 1992), 3SR, the self-sustained sequence reaction (Guatelli et al., 1990), RCA, rolling circle amplification (Fire et al., 1995; Lizardi, 2002) and LAMP, loop-mediated isothermal DNA amplification (Notomi et al., 2000). The foregoing methods may include thermal cycling, for example PCR, or they may be isothermal amplification, for example NASBA.

Also known are homogeneous florescence methods for detecting amplified nucleic acid products in solution without opening the reaction container, typically a tube, well or capillary, and without performing additional manipulations. Commonly these methods include nucleic acid binding dyes (U.S. patent 5,994,056), or hybridization probes (for example, U.S. patent 5,210,015 or U.S. patent 5,925,517) or labeled primers (U.S. patents 6,326,145, 5,866,336; Little et al., 1999), or a combination of dyes and probes (U.S. patent 6,835,257). Detection may be end-point, that is, performed at the conclusion of amplification, or it may be real-time, that is, performed multiple times during the course of an amplification reaction (U.S. patent 5,994,056). Hybridization probe systems for homogeneous detection typically rely on energy transfer between labels, either two probe labels (U.S. patent 5,925,517) or a dye and a probe label (U.S. patent 6,833,257).

Particular examples of amplification are known as *in situ* methods, for example, *in situ* PCR. In one example cells are suspended in a solution of PCR amplification reagents (a "PCR cocktail"), and amplification is carried out in a tube or other reaction container (Haase et al., 1990; U.S. Patent 5,436,144). In another example cells or a thin tissue section are adhered to the surface of a glass slide, which is then overlaid with a PCR cocktail, and amplification is carried out in a special chamber mounted on the glass slide (U.S. patents 5,364,790, 5,538,871 and 5,804,383). Various means are utilized to prevent water loss by evaporation during amplification, including mechanical closures and mineral oil. In both formats cell envelopes are rendered permeable to PCR reaction components, including thermostable DNA polymerase, as a consequence of which amplification takes place both within cells and in surrounding liquid. Therefore, homogeneous methods are not suitable for detecting products of *in situ* PCR. Drawbacks of these *in situ* PCR methods are that the processing of the samples can lead both to false-positive results and false-negative results. In addition, post-amplification manipulation is required for detection, which creates a contamination problem. For these reasons *in situ* PCR has not satisfied the obvious potential of being able to examine any gene in any cell (Uhlmann et al, 1998). Several patents by Stapleton disclose *in situ* PCR in agarose gel (U.S. patents 5,188,963, 5,382,511 and 5,451,500). However, evidence from Stapleton and others suggests that in agarose gel nucleic acid templates and particularly amplicons (amplified product, that is, copies of amplified sequences) will migrate during amplification reaction and will migrate or even be washed away during sample preparation and post-amplification detection, which include washing.

Solution amplification with homogeneous detection requires a minimum number of starting template molecules for reliability. See, for example (LeDuc et al., 2002), which reports a lower limit of 500 copies of starting template molecules for detecting variola virus in clinical samples utilizing PCR amplification with homogeneous fluorescence detection by the 5' nuclease process. With solution PCR amplification of even purified templates, it has been reported that fluorescent signal produced from ten or fewer copies of target sequence is often barely distinguishable above background. (Wittwer et al. 1997a). Solution amplification techniques suffer from non-specific synthesis due to mishybridization of primers, a drawback that tends to be exacerbated in multiplex assays, and from a competition between concurrently amplified targets. Also, quantitative solution assays are technically complicated and insufficiently reliable (Freeman et al., 1999).

Amplification of individual nucleic acid molecules in immobilized media potentially overcomes the sensitivity and reliability drawbacks of solution amplification. A gel or other suitable matrix entraps amplification reagents. Nucleic acid templates are dispersed in the medium, which may be a thin layer, such that individual templates (target sequences) are amplified in isolation from one another to form colonies. This technique, referred to as MCT, the molecular colony technique, is disclosed in patents and publications of A.B. Chetverin and others, including U.S. patents 5,616,478, 5,958,698 and 6,001,568; Russian Federation patents 2,048,522, 2,114,175 and 2,114,915; Chetverin et al., 1991; Chetverina et al., 1993; Chetverin et al., 1997; Chetverina et al., 2002; Chetverina et al., 2004; Samatov et al., 2005. The MCT process, also referred to as "polony" amplification when PCR is the amplification reaction (Mitra et al., 1999), has been implemented by Church et al., utilizing at least one primer tethered to the solid matrix (U.S. patent 6,485,944).

When utilized with primer-dependent amplification processes, however, MCT has a drawback that has prevented its widespread adoption as a diagnostic tool. Reportedly, PCR-amplified colonies comprise only about 10⁸ copies of the target sequence (Mitra et al, 1999; Chetverina et al., 2002), so homogeneous florescence detection methods employed with solution amplification, which produces many more copies, are not utilized with MCT. To illustrate why this is the case, we performed several detection experiments described in Examples 2 and 3 of this application utilizing thin gels to achieve a 2-D effect. Whether homogeneous detection was by nucleic acid binding dye or hybridization probe, 10⁸ copies were insufficient for homogeneous fluorescent detection. Thus, when MCT has been utilized with primer-dependent amplification, post-amplification manipulation has been utilized for detection, as described in the several MCT references cited above. We illustrate one possible post-amplification technique in Example 3(c), namely, *in situ* transcription to create multiple (10¹ to 10²) RNA copies of DNA molecules such that the transcribed RNA copies could be detected utilizing adjacently hybridizing probe pairs. The amplification factor (and hence, the maximal content of a molecular colony) of other primer-dependent exponential systems is also typically within 10⁸ (Guatelli et al., 1990; Walker et al., 1992; Lizardi et al., 1998).

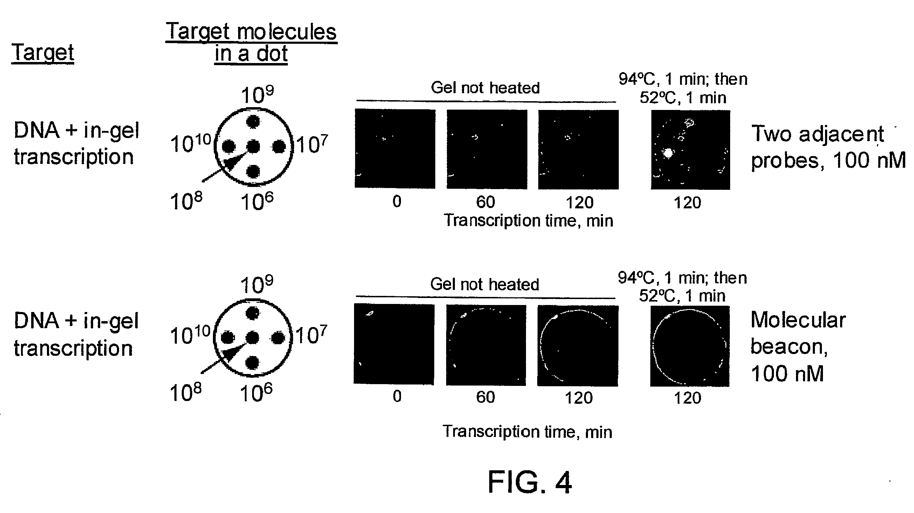

For a homogeneous assay, the most critical parameter is the signal-to-background ratio. Typically, the background in homogeneous systems is much higher than in heterogeneous systems wherein the unbound dye or probe is removed. Even though fluorescence of a probe increases upon binding with target, the background fluorescence may still be unacceptably high because of a higher concentration of unbound molecules. For example, in solution PCR initiated with approx. 8000 target copies, up to 50 ng of a 536-bp (basepair) product (≈10¹¹ molecules) accumulates in a 5-µl volume, which corresponds to a 0.03 µM concentration, whereas the concentration of a probe is 7-fold greater (0.2 µM) (cf. Fig. 4A of Wittwer et al., 1997b). If the fluorescence enhancement upon hybridization of the probe with its target is 10 to 20-fold (as is observed with the best FRET probes), and even if 100% of the PCR product binds the probe, then fluorescence of the sample can maximally increase 2.5 to 4-fold. The actual increase is lower, since the probe can hybridize with only a fraction of target strands because of a competition from the product DNA strands that are complementary to the target.

The estimated product concentration in a DNA colony is much lower than in the above example of the solution PCR. At the end of PCR, the colonies comprising 10⁸ target copies have an apparent diameter of 0.5 to 0.2 mm (Chetverina et al., 2002), which at the gel thickness of 0.4 mm corresponds to a volume of 0.08 to 0.01 µl, and to a DNA concentration of 0.002 to 0.01 µM (that is, 100 to 20 times lower than the concentration of unbound probe), with the expected enhancement of the fluorescence of a FRET probe not exceeding a few percent. This conclusion is consistent with the observation that in solution PCR, the specific signal produced by ≤ 10 copies of a target is often barely distinguishable from background (Wittwer et al., 1997a). In the molecular colony format, every colony originates from just one molecule.

The prior art teaches that a far more favorable signal-to-background ratio is provided by a sequence-nonspecific intercalating dye, such as SYBR Green I, because many molecules of the dye can bind with each DNA molecule and the fluorescence enhancement upon binding is as high as several hundred-fold (Haugland, 2002). However, even in this case, detection of molecular colonies requires that unbound dye is washed away (Mitra et al, 1999; Butz et al., 2003).

"Real time monitoring of DNA colonies growing on a polyacrylamide gel" Analytical Biochemistry, vol. 356 (2006) 300-302, Samatov et al. describes a molecular colony technique including the amplification of nucleic acids in immobilized media.

### SUMMARY

This invention is a method for detecting the presence, in a nucleic acid-containing sample, of at least one nucleic acid target sequence in accordance with Claim 1. The disclosure herein includes nucleic acid amplification utilizing MCT and primer-based amplification methods that include MCT with homogeneous fluorescence detection; that is, amplification of individual nucleic acid molecules (templates or target nucleic acid molecules) in an immobilized medium utilizing primer-dependent amplification systems and homogeneous fluorescence detection of colonies utilizing fluorogenic nucleic acid binding dyes, labeled primers or probes, or a combination of dyes and labeled primers or probes. This disclosure includes immobilized reaction mixtures (reaction media) and kits for carrying out such methods.

This disclosure also includes disposable devices, for example cassettes, having one or more separated reaction container that include thin layers or sheets of gels or other matrices useful for preparing thin layers of immobilized medium, which we refer to as "2-D" immobilized medium, and in which amplification can be performed and homogeneous fluorescence of amplified nucleic acid colonies can be ascertained and measured. Gels in the reaction containers are dried and may contain reagents for amplification.

This disclosure also includes *in situ* assay methods, quantitative and qualitative, in which cells either as individual cells or tissue fragments, are embedded and immobilized in a thin layer of a suitable gel matrix, lysed in place within the gel, and washed with a water-miscible organic solvent to remove components that might interfere with nucleic acid amplification under conditions that cause the target nucleic acid (RNA or DNA) to precipitate. Drying the gel removes the solvent. Thereafter a primer-dependent amplification system and a homogeneous fluorescence detection system are added to the gel to provide separately identifiable colonies of amplified target nucleic acid molecules from the cells, and fluorescence from the detection system is detected, preferably in real time, that is, multiple times during the course of the amplification reaction. Target load per cell or at a location within a cell can be estimated by utilizing the C_{T} (threshold cycle), the thermal cycle at which signal rises detectably above background or, for isothermal amplification, the time at which signal rises detectably above background. Also, during or after amplification in the presence of a homogeneous fluorescence reporter system, a melting curve analysis may be performed. If a gel embeds thin tissue sections rather than individual cells, the resulting colonies of amplified product will indicate cells containing the target. Quantification may include the amplification and detection of a known amount of a control template sequence, for example a nucleic acid sequence that is different from the target sequence but amplifies at a similar efficiency and that is separately detected, as with uniquely colored hybridization probes. Another type of control template is the target nucleic acid sequence itself, added to the gel at identifiable locations through the use of calibration particles to which the control molecules reversibly linked, as by hybridization or through a cleavable linker, where the linker is cleaved prior to amplification.

The disclosure further includes non-ordered 2-D arrays of individual intact cells or microscopic particles, or both, embedded in a gel matrix and methods for preparing such arrays. In particular, the disclosure includes a merged gels method, which enables individual cells to be easily arranged as a monolayer just beneath the gel surface. In addition to cells, the merged gels method can be used for arraying a variety of microscopic particles, such as nanoparticles, subcellular organelles, ribosomes, emulsion droplets, paramagnetic beads, etc. The density of cells or particles in an array can vary depending on its use. A high density cell array, in which cells are tightly packed in a monolayer, can contain up to 100 million prokaryotic cells or up to 1 million eukaryotic cells per square centimeter. Such 'Megacell' and even 'Gigacell' arrays can provide for an unprecedented performance of the analysis of individual intact cells, which can be used, for example, for rapid screening of very large genetic libraries. For screening, the arrayed cells can be fluorescently labeled (for example, by a fluorescent antibody that binds with a 'receptor' protein on the cell surface, by a fluorescent protein that is synthesized within the cell, or by a homogeneous fluorescent reporter systems that binds with a nucleic acid within the cell, such as by a molecular beacon probe). The 2-D arrangement permits easily manipulating embedded cells. To allow cells to breathe, the viewing side of the chamber is preferably a material permeable for gases, such as oxygen and carbon dioxide (a non-permeable side may be replaced with a sheet made of such a material after the gel has formed). The composition of the liquid phase of the gel, the gaseous phase surrounding the chamber, and the temperature can be controlled to either allow or not allow the embedded cells to divide. Also, various agents can be introduced into, or removed from the gel by soaking, and the effects of these manipulations can be registered. The agents can be applied either evenly across the gel surface, or in a gradient, to study the concentration effects. The arrayed cells could be inoculated with desired substances using micromanipulators or liposomes, or transformed with plasmids or viruses. A preferred cell array has the shape and size of a digital video disk (DVD), and could be scanned with a DVD drive modified to become a laser fluorescent scanner, preferably, equipped with a confocal optics. One surface of such a disk would accommodate a gel having capacity of up to 10¹⁰ prokaryotic cells (10 Gigacell) or up to 10⁸ eukaryotic cells (100 Megacell), whereas the opposite surface would function like a formatted DVD having a similar capacity. This would provide for recording the exact location of each individual cell, and would make it easy to precisely return to that cell later at any time - like the heads of a DVD drive find any particular byte of any file saved on the disk.

The cells in an array may be inspected while being intact, or they can be lysed to release high molecular weight components. For example, nucleic acids can be isolated in situ according to methods of this disclosure. If desired, the nucleic acids can be further amplified in situ, and their amount within cells can be assessed by the methods of this disclosure or otherwise. In particular, this approach can be used for diagnostics, for example, for detection and analysis of rare malignant cells among a huge amount of healthy cells.

The high density cell arrays can be used for in situ sequencing of nucleic acids. In one example of this disclosure, DNA fragments to be sequenced are ligated into a vector (for example, a single-stranded DNA phage, such as M13, or a plasmid) and amplified within arrayed E. coli cells (with or without in situ cell multiplication). After in situ isolation of nucleic acids, the entire or partial sequence of each of the ligated fragments can be determined by a known in situ sequencing protocol (for example, Shendure et al., 2005). Up to 10¹⁰ different fragments can be sequenced simultaneously. In effect, this could provide for the sequencing of the entire human or like genome on a single DVD-sized gel.

Methods according to this disclosure utilize MCT, amplification of nucleic acids in an immobilized medium to form colonies of an amplified product or multiple amplified products. An immobilized medium comprises a gel or other suitable matrix having within it a nucleic acid amplification system and, for use in this invention, a homogeneous fluorescent reporter system. There is no free liquid phase that is not immobilized, as that would permit migration of templates and products.

Suitable matrices are known in the art. See, for example, Chetverin et al. U.S. patent 6,001,568. Matrices disclosed in that patent include agarose, polyacrylamide, nylon, gelatin, alginate, carrageenan, cellulose, silica gel, titanium sponge, dextran and polyethylene glycol. Suitable matrices have average pore sizes ranging from 100 µm to 5 nm to entrap the liquid phase. For use in this disclosure the matrix must be able to withstand temperatures utilized during the chosen amplification process. For example, a typical PCR amplification may include a denaturation (or strand-melting) temperature of 95°C, and a gel matrix used with that amplification reaction should not become a sol when heated to 95°C, preferably 100°C.

Preferred matrices for use in this disclosure are gels of polyacrylamide, polyacrylamide analogs or derivatives of polyacrylamide and its analogs. For particular applications the gel matrix may be modified with certain substances, such as oligonucleotides, for example, amplification primers, hybridization probes, or capture probes tethered to the gel. The gel may also be modified by the inclusion of charged chemical moieties that retard diffusion of amplified nucleic acid products through the gel.

Except for *in situ* methods, we prefer that a gel or other matrix be prepared in advance as a dried thin sheet from which pieces of appropriate shape and size may be cut, or as a dried thin sheet cast directly into an amplification reaction chamber, such as a well or cassette of this disclosure. Preferably the gel or other suitable matrix is covalently attached to the surface of the reaction chamber. For use in this invention, a dried gel matrix must be capable of absorbing aqueous liquid and swelling to essentially its original volume. We have shown that dried gels can be stored successfully for significant periods of time. For example, we have shown that the polyacrylamide gel of Example 1 retained its properties when stored in a closed container at room temperature for at least 1.5 months.

An immobilized medium can be prepared by casting a gel with an amplification system, including enzymes, buffer, nucleotide substrates and primers, and optionally a sample containing nucleic acid target molecules, included with the monomer, as disclosed in U.S. Patent 6,001,568. We prefer generally to start with a dried matrix and prepare the immobilized medium by applying the aqueous amplification system and fluorescent reporter system onto the gel, whereby the gel soaks up the amplification and reporter systems and swells to its original volume to create the immobilized medium. Sample that contains or might contain at least one nucleic acid target sequence may be included in the solution of the amplification and reporter systems, or sample can be added separately onto the immobilized medium, as disclosed in U.S. patent 6,001,568. In all cases a free liquid phase is avoided.

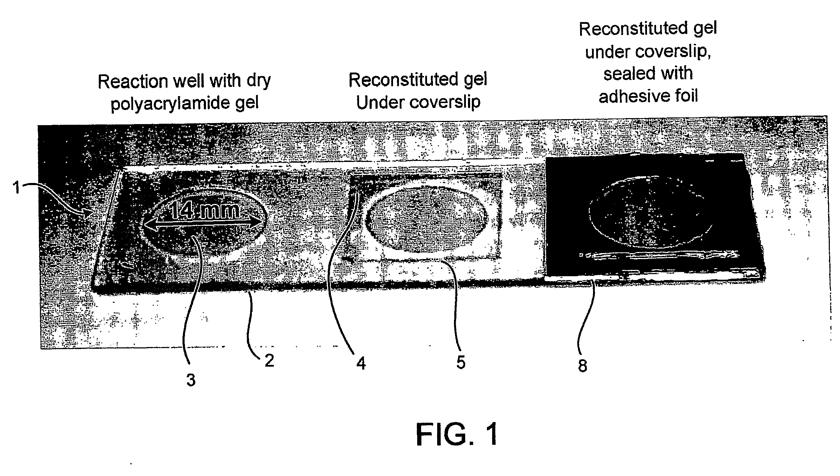

A device containing multiple copies of a suitable reaction chamber for a thin immobilized medium is shown in Figure 1. It comprises a flat base providing good contact with a flat-block heater or thermal cycler, shallow wells for retaining immobilized medium and samples in or on the surface of the medium, a transparent cover for fluorescence detection, and sealing means to seal the reaction chambers prior to the start of amplification.

This disclosure includes a device containing one or a plurality of reaction chambers suitable for performing methods according to this disclosure without contaminating the work environment with amplified products. A suitable reaction chamber comprises a volume that is or can be closed except for inlet and outlet ports, which are themselves closeable. The reaction chamber includes a viewing side, preferably planar, that either is transparent at wavelengths suitable for fluorescent detection or that contains one or more transparent areas, or "windows" for fluorescent detection. Typically the viewing side is the top of the reaction chamber. Opposite the viewing side is a base, also preferably planar, on which is secured a layer of dried, rehydratable gel suitable for use in methods of this disclosure. The reaction chamber is preferably thin, that is, the distance from the viewing side to the base (thickness dimension) is both small, 1 µm to 1 mm, and significantly smaller, by a factor of ten or more, than at least one dimension of the viewing side. If the smallest dimension of the viewing side is at least ten times larger than the thickness dimension, the reaction chamber may be a thin cylindrical slice, a thin rectangular slice or a thin slice having any other desired planar shape, for example, elliptical. If one dimension of the viewing side is large, but the other is small, the reaction chamber will be elongated, that is, rod- or capillary-like, and the 2-D pattern of nucleic acid colonies in the chamber's gel will be more of a 1-D pattern. In the latter case, the reaction chamber may be deformable, as by folding or bending, into rectangular, circular, spiral or other shape to accommodate an amplification instrument. The dried gel layer in the reaction chamber may be sufficient to fill the chamber upon rehydration. For merged gel methods, however, the rehydrated gel layer plus the added second gel will be sufficient to fill the reaction chamber. Put another way, for merged gel methods, the rehydrated dried gel layer may or may not fill the reaction chamber.

The reaction chamber has inlet and outlet ports, preferably one of each, through which liquid solutions can be injected into the chamber across the surface of the gel layer and through which air can escape during such injection. It is desired to eliminate or at least minimize the presence of air bubbles in the filled reaction chamber. To that end sharp corners that trap air should be avoided.

In use the reaction chamber is to be closed, that is, sealed sufficiently to prevent dehydration of an immobilized medium placed therein and sufficiently to prevent escape of nucleic acid amplification products from the reaction chamber to the environment or to other reaction chambers in the device. The ports, therefore, are closable, as by a cap, clamp or plug. Alternatively the ports may be heat sealable or provided with internal adhesive to be pressure sealable. In certain examples the device may be supplied in two (or more) parts, one of which includes the viewing side of the at least one chamber and the other of which includes the base of the at least one chamber. These examples are preferred for devices for QISA methods, as they permit lysing and washing before the at least one reaction chamber is closed. In these examples the part that includes the viewing side is closeable onto the other side so that during amplification the chamber is sealed as described above.

Primer-dependent amplification systems useful in this disclosure to amplify a target nucleic acid sequence that is or may be present in a sample are cell-free and enzyme-based exponential amplification systems. They include reagents for performing a polymerase chain reaction (PCR). PCR methods and systems are well known. See, for example, U.S. patents 4,683,202 and 4,965,188. The amplification system may include means to suppress the amplification reaction until it is desired to begin, for example, a "hot start enzyme." If the target nucleic acid sequence to be detected is RNA rather than DNA, a DNA amplification system may include reagents for reverse transcription. PCR methods useful in this disclosure include not only symmetric PCR utilizing primers in equal concentration, but also asymmetric PCR methods that produce largely single-stranded products by utilizing primers in differing concentrations (U.S. patent 5,066,584), including an advanced asymmetric PCR method called LATE-PCR, linear after the exponential-PCR (Sanchez et al., 2004; Pierce et al., 2005).

Other primer-dependent amplification systems are also useful in this disclosure. Such systems include reagents for performing any of the following amplification reactions: NASBA, nucleic acid sequence-based amplification (Compton, 1991); 3SR, self-sustained sequence replication (Guatelli et al, 1990), SDA, strand-displacement amplification (Walker et al., 1992; Dattagupta et al., 1995), TMA, transcription-mediated amplification (U.S. patents 5,399,491 and 5,554,516), RCA, rolling circle amplification (Fire et al., 1995; Lizardi, 2002) and LAMP, loop-mediated isothermal DNA amplification (Notomi et al., 2000). NASBA, 3SR, SDA, TMA, RCA and LAMP are isothermal nucleic acid amplification methods.

Methods, immobilized reaction mixtures and kits according to this disclosure utilize homogeneous fluorescence detection. Homogeneous detection methods are detection methods that do not require opening an amplification reaction mixture during or after the amplification reaction in order to perform detection. Homogeneous fluorescence methods produce a change in fluorescence indicative of amplification. Homogeneous fluorescent detection systems useful in this disclosure utilize nucleic acid binding dyes, fluorescently labeled hybridization probes, fluorescently labeled primers, and combinations of nucleic acid binding dyes with fluorescently labeled probes or primers. Nucleic acid binding dyes, including dyes sometimes referred to as intercalators and dyes referred to as groove binders, are generally useful in this disclosure as indicators that double-stranded products have been produced (Haugland, 2002; Bengtsson et al., 2003; Neidle, 1994; U.S. Patent 6,902,900). Detection of fluorescence from nucleic acid binding dyes can be performed after the completion of amplification (end-point detection at a fixed temperature or generation of a melting curve) or multiple times during the course of amplification, which is referred to as "real-time" detection (see, for example, U.S. Patent 5,994,056; Di Gusto et al., 2005). Nucleic acid binding dyes can be rendered sequence-specific by linking them to oligonucleotides capable of sequence-specific hybridization with amplification products.

Homogeneous fluorescence detection useful in methods of this invention also include the use of probes and primers that are fluorescently labeled and whose signaling depends on hybridizing to their target or, in some cases for primers, being incorporated into an amplification product. Homogeneous primer and probe systems rely on energy transfer. Energy transfer can occur between two fluorophores, between a fluorophore and a nonfluorescent chromophore (quencher) or between a nucleic acid binding dye and any of the foregoing. Energy transfer is most generally by a phenomenon known as FRET (fluorescence resonance energy transfer), but non-FRET transfer can also be utilized (U.S. Patent 6,150,097).

Immobilized medium is prepared by immobilizing a primer-dependent amplification system and a homogeneous fluorescent reporter system in a suitable matrix. Nucleic acid molecules to be amplified are introduced into the medium during its immobilization or subsequent to its immobilization. If the matrix is a gel, the amplification system or the homogeneous fluorescence detection system or the nucleic acid targets of amplification or any combination of the foregoing can be incorporated in the gel-forming ingredients prior to gelation. Alternatively, one or more of them may be spread across an already formed matrix so as to be taken up by the matrix such that no free liquid remains in the finally constituted immobilized reaction mixture. Preferred examples utilize a thin layer of immobilized medium so that colonies of amplified individual nucleic acid molecules or clusters occur in the thin layer, which we refer to as 2-D medium (if elongated, the 2-D medium is sometimes referred to as a 1-D medium), for detection either in real-time during amplification or in an end-point detection following the completion of the amplification reaction.

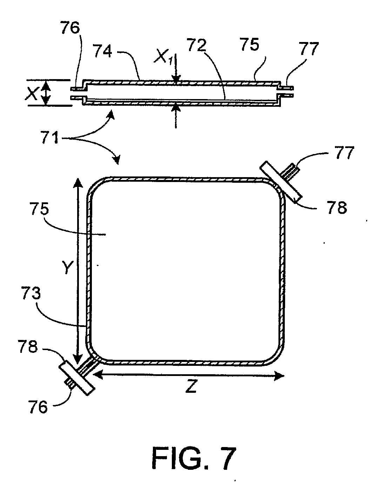

Cell arrays according to this disclosure are prepared by including a suspension of cells in the polymerization mixture for forming the gel, and the cells become embedded in the gel produced by the polymerization reaction. Preferably the gel-forming procedure immobilizes cells in a monolayer just beneath the gel surface for two reasons: first, to ensure access of amplification enzymes to each cell, and second, to position colonies of amplified targets in the same focal plane for ease and reliability of detection. Positioning of the cells can be achieved by a density differential between the polymerization mixture and the cells so that the cells collect at one side (float) or the other (sink) of the gel as it forms. Alternatively we utilize a merged-gel procedure in which a slide (Figure 1) or reaction chamber (Figure 7) is first prepared with a film of a first dried gel on one surface. Then the cell-containing polymerization mixture is added, swelling the first gel and producing merged gels having cells concentrated in a layer beneath the surface of the resulting gel. The two gels may be chemically the same or different.

Methods, reaction media and kits according to this disclosure also include lysing of cells that are immobilized in a gel matrix as the means to provide nucleic acidacids for in situ sequencing or for amplification in immobilized medium. We refer to the latter method as QISA, quantitative in situ assay, because it enables quantification.

Embedded cells are lysed and washed in place. Lysing is performed using a lysing solution that ensures substantially complete lysis and that substantially preserves the integrity of the nucleic acid or nucleic acids to be amplified. Lysing solutions based on GTC (guanidine thiocyanate) or on SDS (sodium dodecyl sulfate) have been shown to be particularly effective for E. coli cells, but the procedure is not limited to any particular lysing solution. Certain examples may include enzymes (lytic enzymes, deoxyribonuclease, ribonuclease), which are inactivated prior to addition of the amplification and detection systems. The gel is then washed to remove substances capable of inhibiting subsequent target nucleic acid amplification, that is, lysing reagents and substances originating in the cellular sample. Washing is performed with a water-miscible organic solvent under conditions causing the target nucleic acid (DNA or RNA, or both) to precipitate. Preferred solvents are lower alcohols, but other solvents and solvent mixtures, of which a number are known in the art (for example, U.S. Patent 5,945,515) may be used. Preferred washing also includes the use of a soluble salt that aids nucleic acid precipitation, such as a chloride, acetate or perchlorate of sodium, ammonium or lithium. Because a high salt concentration may inhibit amplification, a final salt-free wash is preferred. Washed gel is dried to remove solvent. An amplification system and a homogeneous fluorescent reporter system are then added by soaking in to have no free liquid. Amplification and detection proceed as described earlier.

QISA is useful not only for amplifying single nucleic acid molecules into discrete colonies, but also for amplifying clusters of nucleic acid molecules that may reside in a cell or even in a small, thin tissue fragment. Colonies of nucleic acids thus may be either larger than a cell or smaller than a cell, in which case density and distribution of particular DNA and RNA molecules within a cell can be ascertained.

For accurate quantitation of local target concentrations, cellular, multi-cellular or sub-cellular, reaction media and kits preferably include at least one positive standard which is preferably but not exclusively added to the same gel as the target. Each standard contains a known number of amplifiable nucleic acid molecules that serve as templates for amplification. As a standard one can use individual molecules of a template having a different nucleic acid target sequence, and therefore a target of a separate probe, but having amplification properties very similar to those of the target being analyzed. Alternatively, one can use calibration particles, for example glass beads, to which are linked, covalently or non-covalently (as by hybridization, for example) a known number of template molecules, which in this case may be a different target sequence as described above or may even be the target being analyzed. If the template is the target sequence, the location of the calibration particles is made identifiable. Local concentration is determined by comparing C_{T} values for the target sequence clusters and for clusters arising from the standard or standards.

The details of one or more examples of the invention are set forth in the accompanying drawings and the description below. Other features, objects, and advantages of the invention will be apparent from the description and drawings, and from the claims.

### DESCRIPTION OF DRAWINGS

Figure 1 shows a microscopic slide with three reaction wells used in experiments disclosed in the examples of this disclosure.
Figure 2 shows fluorescent images of a gel of Example 2, taken after the indicated PCR cycles, demonstrating an attempt to visualize molecular colonies by homogeneous staining with SYBR Green I.
Figure 3 demonstrates attempts to visualize, by homogeneous staining with adjacently hybridizing FRET probes, the indicated number of DNA or RNA molecules applied to a gel as miniature aliquots.
Figure 4 demonstrates attempts to visualize, by homogeneous staining with adjacently hybridizing FRET probes or with a molecular beacon probe, the indicated number of DNA molecules applied to a gel as miniature aliquots and then transcribed *in situ.*
Figure 5 demonstrates images of molecular colonies obtained during in-gel PCR in the presence of adjacently hybridizing FRET probes, of a molecular beacon, or of a combination of SYBR Green I and a fluorescent probe capable of accepting the energy emitted by the dye.
Figure 6 shows a "fluorescence intensity" versus "PCR cycle number" plot obtained for the encircled molecular colony.
Figure 7 shows, schematically, a reaction chamber according to this disclosure.
Figure 8 shows images of molecular colonies generated by clusters of the indicated number of target molecules, obtained during in-gel PCR in the presence of adjacently hybridizing FRET probes.
Figure 9 shows an ethidium bromide-stained agarose gel demonstrating the pattern of DNA and RNA released from *E. coli* cells upon lysis with the indicated reagents.
Figure 10 shows an ethidium bromide-stained polyacrylamide gel demonstrating the fragment pattern of restriction endonuclease HpaII-digested plasmid pUC129 extracted with a saline alcohol obtained by mixing 45 volumes of ethanol with 55 volumes of a solution containing the indicated concentration of ammonium acetate.
Figure 11 demonstrates images of molecular colonies generated by the indicated number of *E*. *coli* cells embedded and lysed within a polyacrylamide gel, obtained during in-gel PCR in the presence of adjacently hybridizing FRET probes.

Like reference symbols in the various drawings indicate like elements.

### DETAILED DESCRIPTION

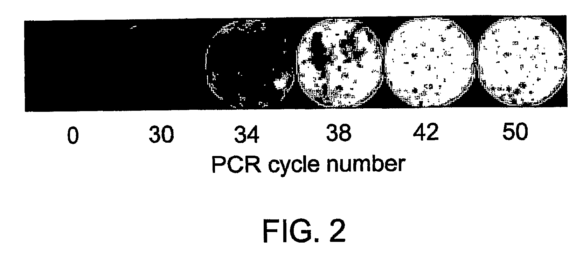

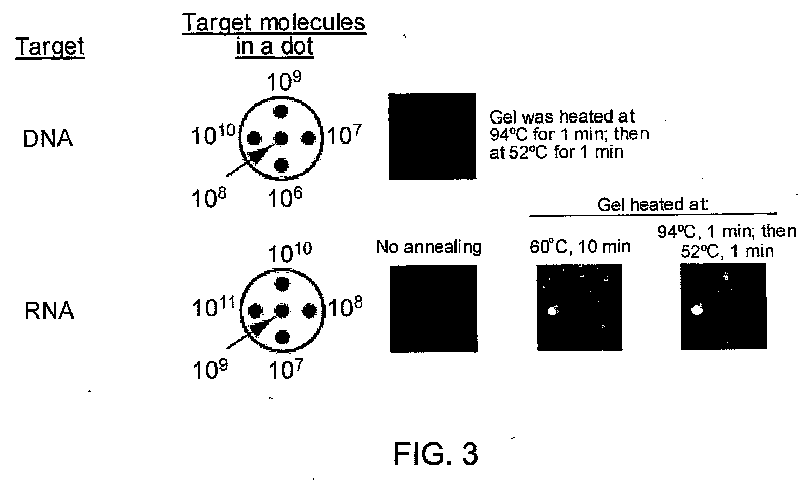

The prior art teaches that methods for primer-dependent amplification of nucleic acids in an immobilized medium tend to produce only about 10⁸ copies of amplified product per colony, an amount generally insufficient for homogeneous fluorescence detection. This problem is illustrated below in working Examples 2-3 and accompanying Figures 2-4. Example 2 reports primer-dependent amplification (specifically, PCR) by MCT, the molecular colony technique, in the presence of a DNA binding dye (specifically, SYBR Green I) utilizing the apparatus shown in Figure 1. Scanned images of the gel after several numbers of PCR cycles (0, 30, 34, 38, 42 and 50), shown in Figure 2, reveal that as the number of cycles increased, the gel's general level of fluorescence increased, but discrete colonies of amplified product did not become visible. This result illustrates the teaching in the art that unbound dye must be washed away in a non-homogeneous process (Mitra et al., 1999; Butz et al, 2003). Example 3 provides additional illustration. Varying known amounts (10⁶, 10⁷, 10⁸, 10⁹ and 10¹⁰ copies) of a target nucleic acid fragment were spotted on a gel in the pattern shown in the top row of Figure 3. Then the gel was soaked in a solution containing adjacently hybridizing FRET probes, heated to melt apart the target strands, and brought an annealing temperature to 52°C for the probes to hybridize. The result, shown in the top row of Figure 3, was that none of the spots became visible. The experiment was repeated, but this time the DNA template was first transcribed to mRNA by incubation with T7 RNA polymerase before the spotting. Varying amounts (10⁷, 10⁸, 10⁹, 10¹⁰, 10¹¹ copies) of the mRNA were spotted on gel in the pattern shown in the bottom row of Figure 3 before the gel was soaked in a solution containing adjacently hybridizing FRET probes. The gel was then scanned immediately and then after annealing of the probes, with or without a prior heating step. Results either way, shown in the bottom row of Figure 3, were that spots having 10¹⁰ and 10¹¹ copies of the target mRNA became visible after annealing, but spots with 10⁹ or fewer copies did not. We also combined simulated DNA amplification (spotting the gel with varying amounts (10⁶, 10⁷, 10⁸, 10⁹, 10¹⁰ copies) of DNA fragment according to the pattern shown in Figure 4), coupled with *in situ* transcription by including T7 RNA polymerase in the probe-containing hybridizing solution. This last method is not suitable for real-time monitoring. Moreover, the heat-labile RNA polymerase has to be introduced after PCR which requires post-amplification manipulations of the gel, and the gel must be heated to provide for probe hybridization which inactivates the RNA polymerase. Although an unsuitable method for that reason, the extra amplification provided by a two-hour transcription incubation did produce sufficient amplified target from 10⁸ spotted DNA molecules for detection with a pair of adjacently hybridizing FRET probes (Figure 4, top row) but not with a molecular beacon probe that we also tested (Figure 4, bottom row).

We have discovered that combinations of a primer-based amplification system and an appropriate low-background homogeneous fluorescent reporter system can be used in homogeneous assays, real-time or end-point, with nucleic acid amplification in an immobilized medium, preferably a thin gel layer, in which the target nucleic acid molecules reside in a layer near a surface for 2-D (including elongated 2-D, that is, 1-D) detection. From teachings in the art and from our own experiments described above, it was surprising to discover that primer-based nucleic amplification could be combined with homogeneous fluorescence detection in methods, reaction media and kits suitable for homogeneous assays, including real-time assays, to detect a single nucleic acid molecule in an immobilized medium. Techniques for doing this include using gels and other matrices suitable for nucleic acid amplification in thin solid media appropriate for 2-D imaging; using primer-based amplification systems such as PCR; and using energy-transfer detection systems, for example FRET, with nucleic acid binding dyes and labeled primers and probes for detecting the product of the chosen amplification system in a colony originating from a single nucleic acid target molecule and containing reportedly no more than 10⁸ target copies.

We are not certain why this works. We theorize that in a growing molecular colony, a nucleic acid product of amplification may have an uneven concentration, or that dye and probes from the surrounding area diffuse into a colony to improve signal-to-background ratio, or perhaps both. However, we do not wish to be bound by any theory of operation. The several systems that we illustrate in the Examples provide sufficient guidance for the construction of satisfactory methods, reaction media, and kits. Examples of combined amplification and detection systems according to this disclosure that we demonstrate in the Examples include: a symmetric PCR amplification system with a pair of adjacently hybridizing FRET probes and detection by exciting the donor probe and monitoring fluorescence from the acceptor probe; an asymmetric PCR amplification system with the same detection system and procedure; an asymmetric PCR amplification system with a molecular beacon probe and detection by exciting and monitoring the probe's fluorophore when separated from the probe's non-fluorescent quencher; an asymmetric PCR amplification system with a nucleic acid binding dye and a labeled probe capable of receiving energy from the dye by FRET, and detection by exciting the dye and monitoring fluorescence from the probe; and an asymmetric PCR amplification system with a fluorogenic nucleic acid binding dye and a labeled probe capable of receiving energy from the dye by FRET, and detection by exciting and monitoring the dye.

Amplification and detection in an immobilized medium is performed in the Examples in a reaction chamber that prevents evaporative loss from an aqueous system. A device containing three satisfactory reaction chambers that we utilized in amplifications reported in the Examples is shown in Figure 1. Device 1 comprises a glass microscope slide 2 containing a series of shallow wells 3 that are 14 mm in diameter and 0.4 mm deep. A transparent cover slip 5 is securable to slide 2 of each well 3 by a ring of mineral oil 4 around the well. Together a well 3 and a cover slip 5 create a 2-D reaction chamber. Device 1 also includes a sealing strip 6 of adhesive foil that is placed over each reaction chamber prior to the start of amplification. Foil strip 6 has a portion removed corresponding to the location of underlying well 3 to permit fluorescence detection through the cover slip.

As stated above, this disclosure includes a device containing one or more reaction chambers suitable for the performance of the amplification and detection methods of this disclosure. An example of a device comprising a single reaction chamber is shown in Figure 7. Referring to Figure 7 the device, cassette 71, is shown in plan view and cross-sectional elevation. Viewing side 75 is transparent and permits excitation and detection of fluorescence from an underlying immobilized medium. Opposite viewing side 75 is base 74. Base 74 has secured to its inner surface rehydratable dried gel layer 72; in other examples, the gel layer can be secured to the viewing side or be not secured at all. The reaction chamber is thin for a 2-D presentation of amplified products. Dimension Xi, which we refer to as the thickness dimension, is small. It can be as small as 1 µm or as large as 1 mm. Dimensions Y and Z are both more than ten times larger than dimension Xi. Other examples have one of the latter dimensions small, not unlike Xi, and the other large to create a narrow, elongated reaction chamber, for example a rod or capillary. Placed oppositely across the reaction chamber 71 are ports 76, 77 through surrounding wall 73, which joins viewing side 75 to base 74. Ports 76, 77 are closable by clamps 78. When liquid is injected through one port, gel 72 rehydrates and fills the entire reaction chamber, whereas displaced air leaves the chamber through the other port. To prevent or at least minimize entrapment of air bubbles, surrounding wall 73 has rounded corners.

It is understood that the larger are dimensions Y and/or Z of the reaction chamber, the greater is the number of colonies of amplified products that can be viewed as individual colonies. For some applications, a chamber whose one dimension is substantially larger than the two others might be preferable. In such a rod-like (or capillary-type) chamber, colonies would be arranged along the larger dimension in a substantially linear 1-D pattern. If the capillary is flexible, it can be folded or wound in a planar cassette to permit accommodation in a standard PCR machine. One advantage of the capillary-type chamber is that after amplification, a piece containing a single molecular colony can be easily excised from the reaction chamber for a further amplification, analysis, or another use of the molecular colony content. It is understood that that use of nonstandard PCR machines, such as air-driven RapidCycler™ (Idaho Technology, Salt Lake City, Utah) may require an appropriate design of the amplification device and chamber or chambers.

The amplification chamber according to this disclosure contains a dry gel, which is capable of swelling upon rehydration and whose chemical and physical properties are compatible with the amplification system and reaction. Many suitable gels are known in the art (Russian Federation patents 2,048,522; U.S. patent 5,616,478). The preferred gel of this disclosure is polyacrylamide gel, its derivative or chemical analog. These gels are essentially hydrophilic polymers resulting from polymerization of ethylene-containing monomer units and include, by way of example, acrylamide, methacrylamide, acrylic acid, methacrylic acid, and structurally related esters of the foregoing as described in U.S. Patent 5,932,711. Polyacrylamide gels resist multiple PCR cycles and, after being completely dried, restore to their volume, shape and other properties upon soaking in the original amount of aqueous liquid (Chetverina et al., 2002). The gel is preferably attached, most preferably covalently attached, to the chamber inner surface. For example, polyacrylamide gel can be attached during polymerization to a glass surface pre-treated with Bind-Silane A174, as disclosed in Example 1. The amount of the gel should be sufficient to ensure that the entire chamber volume is filled prior to start of the amplification reaction. If only a dried gel is utilized, the amount is sufficient to fill the chamber after the gel swells upon soaking in the aqueous reaction mixture injected into the cassette. (To prevent migration of amplified nucleic acids, no unabsorbed liquid should remain in the chamber during amplification reaction!) Preferably, before the gel is dried prior to or during device preparation, it should have the same thickness, shape and volume as it will have after swelling up within the reaction chamber. If a second gel is to be cast atop the dried gel, the amount of the merged gels is sufficient to fill the reaction chamber. During manufacturing, the gel can be cast, washed (if necessary) and dried outside of the reaction chamber or chambers on a flat, impermeable sheet. The size of the sheet can be larger than is required for one chamber, and it can be cut into appropriate pieces before assembling devices. The gel-coated sheet or a piece of the gel-coated sheet can comprise a reaction chamber side, or it can be attached (by gluing, sealing or otherwise) to the inner surface of a chamber side. For some applications, the gel matrix may be modified with certain substances, such as oligonucleotides. One can include in the polymerization mixture for a gel matrix Acrydite™ (Mosaic Technologies, Inc., Boston, MA)-modified oligonucleotides (U.S. Patent 5,932,711) that can function, for example, as amplification primers (U.S. Patents 5,641,658 and 6,485,944), as capture probes or as hybridization probes, including fluorescent probes. At least one primer can be tethered to the matrix, in which case at least one amplification product strand will be covalently immobilized (U.S. Patent 6,485,944). The matrix can be modified with charged groups such as positively charged secondary, tertiary or quaternary amine groups, which can further retard the diffusion in the gel of amplified nucleic acids by reversibly interacting with them (U.S. patent 5,616,478).

The material of the device should meet the following requirements: (1) good heat conductivity of the side (base) 74 contacting the heating block or another heating (cooling) source, depending on the design of a PCR thermal cycler or other amplification instrument; (2) heat resistance at up to the highest temperature to be encountered in use (typically 100°C if the cassette or other device is to be used for PCR); (3) low capacity to adsorb proteins and nucleic acids; (4) the surface on which any gel 72 is cast in place should not interfere with gel polymerization; (5) at least a part of the cassette walls (a window) 75 should be transparent for the excitation and emission wavelengths which are to be used for monitoring colonies. Suitable materials are glass, certain transparent plastics and, except for the transparent side, certain metals and ceramics. Even unsuitable materials could be used if they have the required temperature resistance and their surface is modified to meet the above requirements. Reaction chambers can be of any shape, such as round, elliptic, or rectangular; in the latter case, the corners should preferably be rounded to avoid entrapment of air bubbles.

Referring to Figure 7, cassette 71 is equipped with at least one inlet port 76 and at least one outlet port 77 for each reaction chamber, for injection of the reaction mixture and for outflow of the displaced air, respectively. The design of the ports should permit their tight closure by a plug, a clamp 78 or another sealing means after filling in the cassette with reaction mixture, to prevent drying of the gel during amplification reaction and contaminating the environment with amplified nucleic acids. The Hyb-Seal™ (MJ Research) and HybriWell™ (Grace Bio-Labs) chambers possessing two ports can be modified to prepare a cassette of this disclosure by securing to one major surface a film of a dry gel.

One device may contain more than one amplification chambers. A multi-chamber cassette may be useful for applications in which serial dilutions of a sample are to be tested; it may also be useful when a number of samples are to be run in parallel (for example, an experimental sample and a control sample with which the first is to be compared). Wherein serial dilutions are to be assayed, the multi-chamber device may be equipped with a microfluidic device capable of automatically preparing such dilutions during injection of a sample.

The device may be included as a part of an assay kit of this disclosure. It may be manufactured and distributed either completely or not completely assembled. For example, a cassette may comprise an open-top chamber and a separate viewing lid 75, from which a user assembles a complete cassette by loading dried-gel layer 72 and sealing lid 75 over the open-top chamber. An unassembled cassette may be useful for preparing cell arrays and for *in situ* assays (discussed below) in which the gel is to include embedded cells, tissue fragments and, in some examples, control particles at identifiable locations.

As indicated above, primer-dependent exponential amplification systems and methods are useful in this disclosure. Such methods may be isothermal, for example NASBA, or they may include thermal cycling, for example PCR. They may produce single-stranded amplification product to be detected or they may produce double-stranded amplification product. DNA amplification methods such as PCR are adaptable to RNA targets by including a reverse-transcription step utilizing reverse transcriptase or a DNA polymerase with reverse transcriptase activity (U.S. Patent 5,407,800). A PCR system may include "hot start," for example, a chemically modified DBA polymerase (U.S. patent 5,673,152), an antibody-bound DNA polymerase (U.S. patent 6,338,671) or an aptamer (U.S. patent 6,020,130). Methods, such as symmetric PCR, that produce double-stranded amplification product exponentially using a pair of primers, may include a final reaction portion that produces single-stranded product by linear amplification with one primer. This technique, referred to as asymmetric PCR, is accomplished by having one primer present in the amplification system in excess relative to the other primer. Asymmetric PCR, as used here and in the appended claims, includes PCR methods in which one primer is added at a molar concentration higher than the other, and includes the PCR method known as LATE-PCR (Sanchez et al., 2004; Pierce et al., 2005).

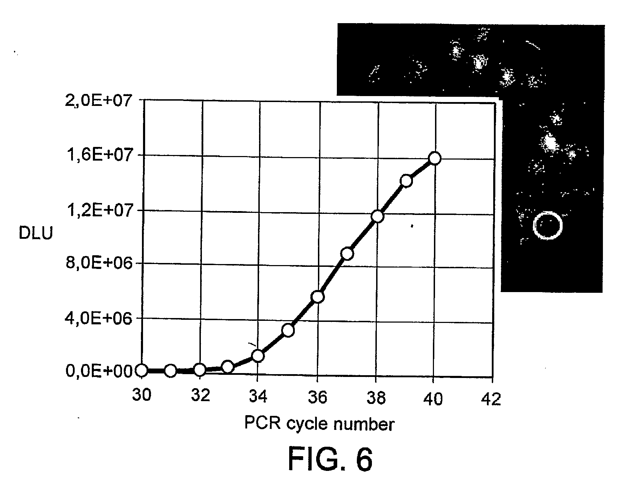

For homogeneous detection according to this invention, the immobilized reaction mixture that includes the amplification system and sample also includes an appropriate homogeneous fluorescent reporter system. A reporter system is appropriate if, when combined with the chosen amplification system, it is able to provide a detectable signal above background for amplified product in the immobilized medium by the completion of amplification starting with a single target nucleic acid (RNA or DNA) molecule. For real-time detection such a signal is preferably produced significantly before completion of the amplification reaction so as to enable preparation of a curve of signal versus time of reaction or, if amplification includes thermal cycling, cycle number, like one shown in Fig. 6. For PCR methods, for example, we prefer that a signal above background be detectable at least four, preferably at least five, cycles before the signal plateaus so that a curve can be constructed of at least four, preferably at least five, points. In practice, this means that in combination with the amplification system chosen, a detectable signal in a 2-D immobilized medium should arise from a several times lower amount of product than accumulates in a colony by the completion of amplification.

This invention is not limited to any particular homogeneous fluorescent reporter system. We demonstrate several systems. Numerous homogeneous fluorescent reporter systems are available. In each of these methods, fluorescence of a label changes, most generally increases, upon its binding with, or incorporation into, the amplification products. Many fluorescent labels and labeled substances are catalogued in a handbook (Haugland, 2002).

The homogeneous detection systems known in the art use fluorogenic nucleic acid binding dyes, sequence-specific oligonucleotide probes and primers carrying such dyes or carrying fluorogenic groups capable of fluorescence energy transfer, or a combination thereof. Fluorescence energy transfer is generally by FRET (fluorescence resonance energy transfer) between donor and acceptor labels or dyes. In some systems preferred acceptors are non-fluorescent chromophores. Energy transfer other than FRET is also utilized (U.S. Patents 6,150,097 and 6,635,427). Known dyes, probes and methods suitable for homogeneous detection of nucleic acids amplification products include, but are not limited to, those described below.

Nucleic acid binding dyes are classified as intercalators and groove binders. Both have a low fluorescence in aqueous solutions and a substantially higher fluorescence upon binding to nucleic acids, due to displacement of water molecules from fluorogenic groups. Fluorescence of intercalators increases due to positioning between stacked bases of double-helical structures present in the amplification products (such as a double-stranded DNA and hairpins of a single-stranded RNA). Examples of intercalators include, but are not limited to, ethidium bromide, propidium iodide, acridine-derivatives, SYBR Green I (a DNA-specific dye) and SYBR Green II (an RNA-specific dye), and many other intercalators described elsewhere (Haugland, 2002; U.S. Patent 6,902,900). Groove binders are capable of non-covalent binding to the minor groove or the major groove of a double helical structure. Examples of groove binders include, but are not limited to Hoechst dyes, DAPI (4',6-diamidino-2-phenylindole) (Haugland, 2002) and BEBO (Bengtsson et al., 2003). Also known in the art are mixed-type dyes, capable of both intercalating between bases and binding to a groove. Examples of these include, but are not limited to, TOTO-1 (and its parent compound thiazole orange) and YOYO-1 (Haugland, 2002). Further examples of intercalators and groove binders, a discussion of their structural features and the mechanism of their binding to DNA can be found in Neidle, 1994.

Of the nucleic acid binding dyes, the most widely used in homogeneous assays are intercalating dyes, such as ethidium bromide and SYBR Green I. They are used for real-time monitoring of PCR (Higuchi et al., 1992; Higuchi et al., 1993; U.S. Patent 5,994,056) as well as of isothermal amplifications, such as RCA (Di Giusto et al., 2005). The major drawback of dyes is that they bind to nucleic acids essentially nonspecifically, that is, irrespective of the nucleotide sequence. Although sometimes, by registering fluorescence near the melting temperature of the target-specific PCR product, it is possible to discriminate against non-specific products (such as primer-dimers, which are shorter and therefore melt at lower temperatures) (Morrison et al., 1998; U.S. Patent 6,174,670), this feature restricts the utility of dyes as real-time amplification reporters.

Specificity of assays can be increased by covalently linking said dyes to oligonucleotides capable of sequence-specific hybridization with amplification products (U.S. Patents 5,814,447 and 6,902,900). In this format, dyes continue to increase their fluorescence upon binding with nucleic acids, but now their binding becomes sequence-specific. Instead of oligonucleotides, the dyes can be linked to oligonucleotide analogs in which the natural sugar-phosphate backbone is replaced with an unnatural polymer, such as having modified phosphodiester linkages, unnatural sugar moieties, phosphodiester analogues, completely replaced backbone, those with 2'-5' internucleotide linkages and those with α-nucleotide anomers. Numerous examples of such a sort are disclosed in U.S. Patent 6,329,144. Examples of unnatural oligomers, which are capable of correct Watson-Crick base-pairing with a target nucleic acid sequence include, but are not limited to, phosphorothioate, methylphosphonate and borane phosphonate nucleic acid derivatives (Eckstein, 1991; Agrawal, 1993), peptide nucleic acids (PNA) having a polyamide backbone (Nielsen et al., 1991; Egholm et al., 1993; Nielsen and Egholm, 1999), and morpholino oligonucleotides (U.S. Patents 5,142,04 and 5,185,444). In addition to oligonucleotides capable of the Watson-Crick base-pairing with a single-stranded region of a target, there are known examples of oligonucleotides capable of specific interaction with double-stranded structures resulting in the formation of triplexes (U.S. Patent 6,329,144; U.S. Patent 6,403,313). This invention is not limited to any particular technique for using nucleic acid binding dyes.

Preferred homogeneous fluorescent reporter systems for use in this invention utilize transfer of energy between moieties in the system. The moieties may be two fluorophores, a fluorophore and a non-fluorescent quencher, or a nucleic acid binding dye and a fluorophore. The moieties may reside in the same molecule or in different molecules. The most common energy transfer is known as FRET, fluorescence resonance energy transfer. In FRET, the phenomenon of energy transfer occurs between two chromophore groups, of which at least one (energy donor) is a fluorophore whose emission spectrum overlaps the absorption spectrum of the second group (energy acceptor). According to Förster (Förster, 1948), the transfer occurs by a dipole-dipole resonance interaction between the energy donor and acceptor chromophores. The energy transfer can occur if distance between the chromophores is relatively short (within 70Å, about twice the helix repeat distance in base-paired nucleic acids; Cardullo et al., 1988), and the efficiency of transfer is inversely proportional to the sixth power of the distance (Stryer et al., 1967). At 5°C, transfer efficiency is about 60% if donor and acceptor groups are spaced by 8 basepairs in a double helix, and it falls to 4% as the distance increases to 16 basepairs (Cardullo et al., 1988). In fact, FRET can be used to measure intermolecular distances in the 10-to-60-Å range (Stryer et al., 1967). Due to FRET, the donor fluorescence is quenched. The absorbed energy can be re-emitted as light of a longer wavelength if the acceptor group is also a fluorophore. Alternatively, it is dissipated as heat. Methods that detect an increase in re-emission by an acceptor, which may be accompanied by detection of a corresponding decrease in emission by a donor ("positive" FRET) and method that detect an increase in emission of an excited fluorophore due to a reduction in quenching ("negative" FRET) may be employed in homogeneous assay systems useful in this disclosure.

Positive FRET is employed in a system comprising two oligonucleotide probes capable of hybridizing with a target adjacent to each other in a head-to-tail manner, with said head and tail carrying the donor and acceptor fluorophores (Heller et al., 1983; Cardullo et al., 1988; U.S. Patent 4,868,103). We refer to such probes as adjacently hybridizing FRET probes. A sample is illuminated with monochromatic light absorbable by the donor fluorophore, and light emitted by the acceptor fluorophore that serves as a reporter is detected. Signal exceeding the background fluorescence is only observed if the sample contains target molecules (in our methods, products of the amplification of nucleic acid target sequences), if the two probes have hybridized with the target, and if the donor and acceptor groups are within Förster's distance, preferably spaced by 2 to 8 nucleotides along the target molecule (U.S. Patent 6,174,670). A number of fluorophore pairs suitable for positive FRET are known in the art (U.S. Patent 6,174,670; Haugland, 2002), but the lowest background fluorescence is achievable with pairs whose spectral overlap is relatively small, preferably less than 25%, such as a pair comprised of fluorescein and Cy5 (U.S. Patent 6,174,670). In a variation of this approach, the oligonucleotides hybridized with a template immediately adjacent to each other are ligated by a thermostable DNA ligase. This results in an oligonucleotide carrying both the donor and acceptor groups and hence capable of intramolecular FRET even after melting the probe-target hybrid. In addition, the signal can be amplified by performing many ligation-melting cycles. This technique is sensitive to single-base mismatches at the ligated ends and is suitable for single nucleotide polymorphism (SNP) assays. To prevent the ligated oligonucleotides from being extended during PCR, they are made relatively short for their hybridization temperature be substantially lower than the PCR primer extension temperature, and ligation cycles are performed after completion of a sufficient number of PCR cycles (Chen et al., 1998).

Positive FRET can also occur in a system comprising a fluorophore-containing oligonucleotide probe and a fluorogenic nucleic acid binding dye, such as an intercalator. In this case, the dye binds with the double helix formed by the probe-target hybrid, and can serve as either energy donor or acceptor in respect to the fluorophore linked to the probe (Cardullo et al., 1988, U.S. Patent 6,833,257). In Example 6 below we demonstrate a method according to this disclosure in which a dye acts as a donor and the probe's fluorophore acts as the acceptor.

Negative FRET systems are comprised of a donor fluorophore that also serves as a reporter, and its quencher that can be either fluorogenic or non-fluorogenic group. The two groups are linked to a hybridization probe in such a manner that, in the absence of the probe's target, the reporter and the quencher are close together, and fluorescence is low because of energy transfer, for example FRET. Reportedly, quenching can also be achieved by a mechanism different from FRET (U.S. Patent 6,150,097; U.S. Patent 6,635,427), and non-FRET quenching can be utilized in methods and systems of this invention. A number of suitable reporter/quencher pairs are known in the art (U.S. Patents 5,928,862, 5,925,517, 6,150,097 and 6,030,787; Haugland, 2002). Upon interaction of a probe with target, the reporter and the quencher move apart, resulting in increased fluorescence. In the art, there are a variety of specific designs that employ this strategy.

In the simplest format, the reporter and the quencher are attached to the opposite (5' and 3' ends) of a single-stranded oligonucleotide hybridization probe. Since a single-stranded oligonucleotide folds into a compact globule, the ends with a high probability will occur close to each other resulting in quenching of the reporter. Upon hybridization with target, the probe stretches out and the distance between the ends increases resulting in a decrease of the FRET efficiency and, hence, in an enhancement of the reporter fluorescence (U.S. Patent 6,030,787). A variety of more elaborate designs of negative FRET systems differ in a manner how to further increase energy transfer efficiency within unbound probes and to further decrease energy transfer efficiency upon the probe-target hybridization.

Tyagi and Kramer disclose "molecular beacon" probes, that rely on quenching free-floating probes. A molecular beacon is a single oligonucleotide strand which folds into a hairpin whose loop is complementary to a target sequence, and whose proximal ends (held together by the stem) carry a reporter and a quencher. The reporter's fluorescence is efficiently quenched in the hairpin (closed) conformation and is not quenched in the stretched (open) conformation attained when the probe hybridizes with the target (Tyagi et al., 1996; U.S. Patent 5,925,517). The molecular beacon design may be further elaborated by introducing an additional fluorophore in a vicinity to one which is excited. In the closed conformation, fluorescence is decreased by the quencher (negative energy transfer, for example, negative FRET). In the open conformation, the energy from the excited fluorophore is resonance-transferred to the second fluorophore which serves as a reporter (positive FRET). By varying reporter fluorophores with the same excited fluorophore, it is possible to have a variety of fluorescent emissions when excited by the same wavelength (U.S. Patent 6,037,130). A variation of the molecular beacon design is presented by chimeric Scorpion primers, which are primers carrying at the 5' end a molecular beacon hairpin attached to the primer via a link (such as hexaethylene glycol monomer) that cannot be copied by DNA polymerase. In this format, hybridization of the molecular beacon with its target in the new strand being made by extension of the primer is monomolecular, rather than bimolecular reaction (U.S. Patent 6,326,145).

Pitner et al. (U.S. Patent 5,888,739) disclose a negative energy-transfer probe, part of which is capable of folding into a structure known as "G-quartet" or a structurally similar "I-tetraplex" (Rhodes et al., 1995). Such a structure holds together the reporter and the quencher when the probe is not hybridized to its target, resulting in a low fluorescence level.

For a probe that changes in fluorescence due to hybridization with its target, further increase of fluorescence can be achieved by target-dependent probe degradation. The 5' nuclease process utilizes the 5'→3' exonuclease activity of Taq DNA polymerase (as well as of some other DNA polymerases) used for PCR amplification. DNA polymerase hydrolyzes the probe hybridized to a target when it extends a primer hybridized to the same target molecule. This target-specific hydrolysis liberates the reporter to the solution, maximally relieving quenching. Further, the released fluorescing reporter accumulates in the reaction medium; its concentration increases in each PCR cycle and may exceed the concentration of the PCR products, which provides a very sensitive detection method (U.S. Patent 5,210,015). Reporter de-quenching by probe hydrolysis is also used in a system employing "zymogenic" primer, which contains a sequence that is antisense to a DNAzyme capable of cleaving the probe. Synthesis of its complementary copy during PCR generates an active DNAzyme that cleaves the probe (Todd et al., 2000). However, to adapt these methods for the detection of molecular colonies, the reporter portion of the probe should be tethered to gel matrix to prevent its diffusion following hydrolysis. The probes can be tethered using, for example, the Acrydite™ chemistry (see U.S. Patents 5,641,658 and 5,932,711). Because of having the reporter tethered, the gel matrix will continue to fluoresce upon probe hydrolysis at the colony location even after amplification products diffuse out or are washed away; therefore, such a gel can be stored and used as an assay record.

Negative energy transfer probes useful in this invention also include double-stranded probes. Morrison discloses a probe comprising a pair of oligonucleotides which are complementary to one another and also to the two strands of a target sequence, which hybridize to the two probe strands. Both have a reporter at the 3' end and a quencher at the 5' end (or *vice versa*). In the absence of target, the oligonucleotides hybridize to each other, placing the reporters and the quenchers in close proximity, resulting in nearly complete quenching. Alternatively, one of the oligonucleotides has a reporter, and the other has a quencher on the proximal end. When the oligonucleotides hybridize to the target strands, the reporter is no more quenched and becomes fluorescent (U.S. Patent 5,928,862). Another double-stranded probe includes a fluorophore-labeled probe strand and a quencher-labeled shorter strand that prevents the probe from fluorescing unless the target displaces the shorter strand (Li et al., 2002).

Also useful in method and kits of this disclosure are primers that utilize energy transfer, for example fluorogenic Sunrise™ primers (Oncor, Gaithersburg, MD, USA; also called UniPrimers or Amplifluor primers) whose design is similar to molecular beacons, but which are incorporated into the amplification products and hence may label non-specific amplification products as well (U.S. Patent 5,866,336). A variation of these are so-called LUX™ ("lighting upon extension"; Invitrogen, Carlsbad, CA, USA) primers which contain only the reporter group, whereas quenching is due to interaction with nearby guanine or cytosine bases and is achieved by a special secondary structure design (Nazarenko et al., 2002).

Similarly to probes carrying nucleic acid binding dyes, the hybridization probes for both positive FRET and negative FRET or non-FRET quenching assays can be of different chemical nature. These can be DNA, RNA, or mixed DNA and RNA probes. Probes can be prepared using PNA (Ortiz et al., 1998) and, of course, other chemical analog of oligonucleotides, for example 2'O-methyl ribonucleotides and non-natural internucleotide linkages, capable of Watson-Crick base-pairing (see above). Moreover, in addition to a direct chemical coupling, the fluorophores and quenchers can be attached to the probes non-covalently. For example, FRET-based assays can be performed using biotin-modified oligonucleotides labeled by streptavidin conjugated with a fluorescent rare earth metal complex (U.S. Patent 6,403,313).

Although the homogeneous detection systems strategies discussed above were primarily developed for the use with PCR, most of them are applicable to isothermal amplification systems, as exemplified by the use of molecular beacons or like probes for real-time monitoring of NASBA (Leone et al., 1998) and rolling circle (Nilsson et al., 2002) amplification, and molecular beacon-like primers for real-time SDA (Little et al., 1999).

Utilizing reaction container, a thin gel or other matrix, a primer-dependent, enzymatic exponential nucleic acid amplification system, and a homogeneous fluorescent reporter system, all as described above and in the Examples that follow, MCT methods according to this invention comprise the steps of preparing an immobilized medium containing the amplification system, the reporter system and containing nucleic acid target molecules dispersed in a 2-D pattern (now the complete reaction medium) that is free of non-immobilized aqueous liquid, incubating the reaction medium under conditions, isothermal or thermal cycling, that permit amplification, obtaining a 2-D fluorescent image of the medium by exciting and detecting the fluorescent reporter system without opening the reaction container and without washing away unbound dye, probes or primers. Preferred methods of preparing the immobilized reaction medium comprise providing a dried matrix affixed in the reaction container and then applying to the matrix an aqueous liquid phase containing the amplification system and the detection or reporter system. A sample containing nucleic acid target molecules can be included in that aqueous liquid phase or added separately by spreading the sample across the surface of the matrix to prepare the immobilized reaction medium.

MCT methods according to this disclosure include utilizing two or more different hybridization probes (or adjacently hybridizing probe pairs) that differ in sequence and fluorophore labeling to detect the presence of two or more target sequences (or two or more sites in the same target sequence) that are amplified by the same primer pair. MCT methods according to this disclosure also include utilizing two or more primer pairs in the amplification system to amplify two or more nucleic acid target sequences, together with multiple, differently labeled probes or primers to detect multiple target sequences in the sample, so-called multiplex assays. Detection can be real-time or end-point, and may include generation of a melting curve or melting curves.

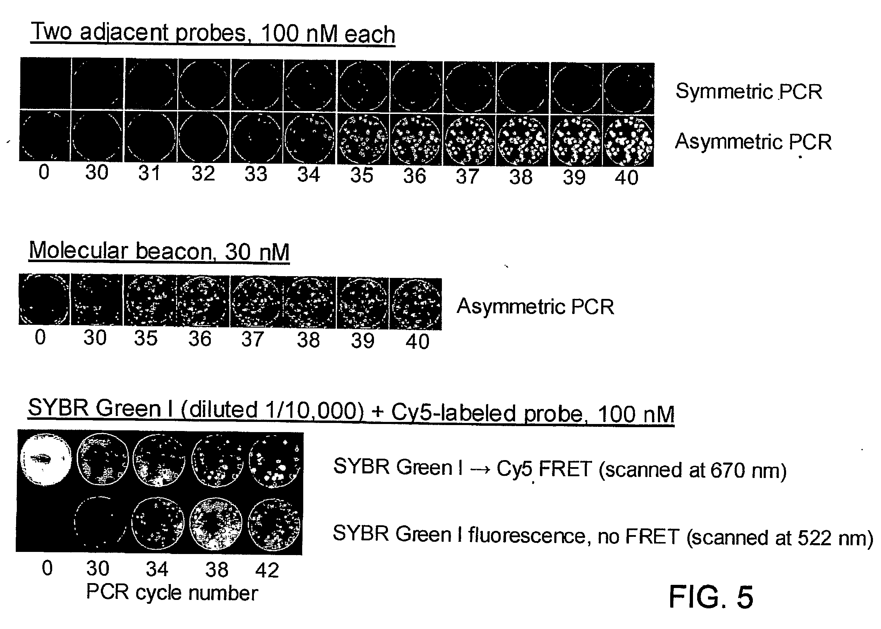

It was unexpected for us to discover that PCR colonies are readily detectable with the same FRET probes that proved inefficient in the "homogeneous" staining of DNA dots (Figures 3 and 4). Experiments were carried out as described in Example 5. Each preformed, dried gel matrix was soaked with a PCR cocktail containing, in addition to the PCR amplification system, a few tens of molecules of a DNA target and the indicated fluorescent detection system. After the indicated number of PCR cycles, a gel was scanned using blue (488 nm) laser to excite fluorescence and an appropriate emission filter, and PCR cycling was continued. The concentrations of PCR primers were either equimolar (symmetric PCR) or adjusted to produce more strands that are complementary to the oligonucleotide probes (asymmetric PCR). The results show that discrete fluorescent spots, whose number approximates the expected number of target DNA molecules in a sample, become visible after the 30th cycle (Figure 5). The time course of the fluorescence produced by an individual colony (Figure 6) is similar to the plot obtained for a real-time solution PCR initiated with ≤10 target molecules (Wittwer et al., 1997a), except that for molecular colonies the noise is smaller, the signal-to-background ratio is higher, and hence the reliability of measurements is better. In a sense, the growth of colonies in a gel is equivalent to a plurality of real-time reactions run in parallel, with each reaction being initiated by exactly one template molecule.

As seen in Figure 5 (top panel), both symmetric and asymmetric PCRs generated colonies that were detected with adjacently hybridizing FRET probes. With symmetric PCR, DNA colonies become visible two cycles earlier than with asymmetric PCR, indicating that amplification is somewhat more efficient. However, with asymmetric PCR, DNA colonies became much brighter, apparently because of the excess of the target strand over its complementary strand. The excess target strand in the amplified product exists in single-stranded form and hence is more available for hybridization with the probes. Asymmetric PCR methods and other methods that yield single-stranded amplified products are particularly preferred for that reason. Molecular beacons also reported amplified colonies (Figure 5, middle panel).

Particularly surprising results were obtained with a sample containing an intercalating dye (SYBR Green I) and an oligonucleotide probe labeled with a fluorophore (Cy5) capable of accepting energy from the dye (Figure 5, bottom panel). The gel was illuminated by blue laser and scanned at two wavelengths, 670 nm (emission maximum of Cy5, upper images) to register the light generated due to positive FRET, and 522 nm (emission maximum of SYBR Green I, lower images) to register the light directly emitted by the dye. The following unexpected observations were made.
(1) At these two wavelengths, the background fluorescence changed in opposite directions as PCR proceeded: it increased at 522 nm and decreased at 670 nm. This indicates that at onset of the reaction, there was a significant nonspecific FRET between SYBR Green I and Cy5 which diminished late in the reaction. The reasons for these effects are not known.
(2) The most significant darkening of the background at 670 nm occurred simultaneously with the appearance of fluorescing colonies, and this made the specific colony fluorescence even brighter.
(3) The increase of the background fluorescence at 522 nm was less pronounced in this experiment than if PCR occurred in the presence of SYBR Green I alone (cf. Figure 2), and this allowed molecular colonies to be detected at this wavelength, too. Although the decrease in the background fluorescence is obviously related to the presence of the Cy5-labeled probe, its mechanism is also not known.

Whatever is the nature of the observed effects, the last experiment demonstrates that molecular colonies can be detected by means of fluorescence generated due to FRET between a nucleic acid-binding dye and a fluorescent oligonucleotide probe, and even by a direct emission from a nucleic acid-binding dye, not mediated by FRET. At the same time, monitoring the FRET-mediated fluorescence is preferable: it is seen that sequence-nonspecific intercalating dye SYBR Green I stains more colonies than the sequence-specific Cy5-labeled probe, suggesting that it stains both specific and nonspecific DNA colonies.

In summary, the results presented in Figure 5 demonstrate that any of the presently known principles of homogeneous fluorescent detection can be used to visualizing molecular colonies.

We theorize that in a molecular colony, DNA is distributed unevenly, so that DNA concentration in the center of the colony is much higher that on the periphery, and it is the colony center that produces most of the signal. It should be noted that this feature is peculiar to the in-gel amplification format, and could not be derived from the previous homogeneous fluorescent detection studies since no concentration gradients are possible in case of amplification in a liquid medium. Another peculiarity of the in-gel amplification format is that a molecular colony is comprised of DNA and/or RNA molecules whose diffusional mobility in a gel is much lower than the mobility of the molecules of a dye or a probe. Therefore, as the colony grows and its mass increases, the dye or probe molecules capable of binding with the amplified nucleic acids may diffuse into the colony from the surrounding gel and accumulate there. This may further improve the signal-to-background ratio, due to both an increase in the concentration of the dye or probe in the colony, and a decrease of their concentration in the surrounding gel. The latter possibility is supported by the fact that the background fluorescence produced by a non-specific FRET between unbound dye and probe decreases synchronously with the appearance of specific colonies (see Figure 5, upper row of the bottom panel).

The results obtained permit the molecular colony diagnostics to become a routine procedure, which can be performed by an ordinarily skilled person. All that is needed is: (1) a reaction cassette containing a dried gel (see above for a proposed construction of the cassette), (2) one or a few solutions that make up the PCR cocktail, including a chosen homogeneous fluorescent reporter system, and (3) a PCR machine with a flat-bed heating block, preferably equipped with a 2-D fluorescent imager employing either a scanning device like that used in this work, or a CCD camera; alternatively, PCR machine and fluorescent imager may be separate devices. The assay procedure comprises: (1) mixing the components of PCR cocktail (including a fluorescent reporter system) and an analyzed sample (a nucleic acid preparation); (2) injecting the solution so obtained into the cassette; (3) waiting until the gel absorbs all the liquid and expands (swells) all over the cassette inner volume; (4) placing the cassette into the PCR machine and starting the cycles; if the machine is equipped with fluorescent imager, it will automatically register and count the colonies and print out the result or store it in a computer; alternatively, the cassette should be at predetermined time(s) inspected with a separate 2-D fluorescent imager; (5) disposing the cassette, including a substantially complete degradation of the amplified targets. If the target is RNA rather than DNA, then the analyzed sample is first subjected to a reverse transcription reaction, either before or after injecting it into the cassette.

Although in the examples provided herein the fluorescent patterns are obtained at a fixed (ambient) temperature following the extension step of a PCR cycle, it is understood that fluorescence can be registered at different temperatures and at different steps of a PCR cycle. If PCR machine is equipped with a 2-D fluorescent imager, it is preferable to continuously monitor the fluorescence generated by molecular colonies. For example, if a sequence-specific oligonucleotide probe is used, the continuous fluorescence monitoring would indicate the temperature at which the probe-target hybrid is melted (or formed). Since melting temperature (Tₘ) decreases by about 4-8°C if a single base mismatch is present, such a melting curve analysis allows even point mutations to be detected if a hybridization probe overlaps a mutation site (U.S. Patents 5,925,517 and 6,174,670). Shifts in the temperatures at which the colony image appears during gel cooling (the annealing step) or disappears during gel heating (the melting step) can be a powerful tool for SNP analysis. Alternatively, the presence of mutations may be detected through melting curve analysis subsequent to amplification.

Of course, besides PCR, other template-directed amplification systems can also be used, such as 3SR (self sustained sequence replication) or NASBA (nucleic acid sequence-based amplification), SDA (strand displacement amplification), RCA (rolling circle amplification), LAMP (loop-mediated DNA amplification), and even Qβ replicase (although the latter is not preferable due to severe background amplification problems). In these cases, PCR machine is not needed. Since all these systems are isothermal and operate at a low temperature, long oligonucleotide probes may hybridize slowly and inefficiently and, hence, there may be problems with real-time detection of growing colonies. If this occurs, the problem could be solved either by decreasing the probe length (to decrease its hybridization temperature to about one at which the reaction takes place) or by heating up the cassette to the hybridization temperature at the end of the reaction (and probably causing denaturing the enzymes), to take a final snapshot of the colony pattern, as is illustrated by Example 3(c) and Figure 4 (top panel) disclosing the detection of products of in-gel transcription.

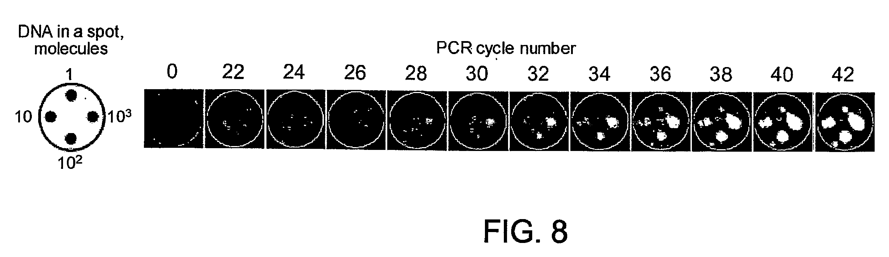

The reported results also provide for another application that can be termed "quantitative in situ assay" (QISA). All colonies in each gel of Figure 5 became visible almost synchronously. This is because each colony originated from a single template molecule. However, if a colony was originated from a cluster of molecules, it would become visible earlier, because lesser number of PCR cycles would be needed to produce the detectable amount of DNA at that location. If in each cycle the number of DNA molecules increased by a factor of 2, then colonies produced by 1000, 100, and 10 clustered template molecules would appear log₂10 ≈ 3.3 cycles earlier than those produced by 100, 10, and 1 molecule, respectively. In reality, the amplification factor is usually somewhat smaller than 2, and the difference is expected to be larger, about 4 cycles. The following simple experiment shows that this is really the case (Example 8 and Figure 8). Miniature droplets of DNA solution expected to contain 1000, 100, 10 and 1 molecules of a GFP-encoding plasmid were applied onto a dry polyacrylamide gel (location of the droplets is schematically shown on the left). After the droplets had dried, the gel was processes as disclosed in Example 4. It is seen that colonies generated by clusters of 1000, 100, 10 and 1 molecules became visible after cycles 26, 30, 34, and 36, respectively, i.e., the time difference in the appearance of the colonies was almost exactly as expected. It follows that by noting the cycle (or, for isothermal amplification, the time of incubation of the reaction medium) at which a colony becomes visible one can estimate the copy number of a DNA template that gave rise to that colony. It should be noted that such a clustered colony differs from a colony produced by a single molecule in that it does not, generally speaking, represent a molecular clone. A similar principle is used in quantitative solution PCR (including real-time PCR); in that case, one can estimate the amount of a target by noting the cycle number at which fluorescence of a test tube reaches certain (threshold) level.

The results of Figure 8 provide for the determination of spatial arrangement and local concentrations of specific DNA and RNA molecules in cells or in tissue slices that have been entrapped in a gel during its polymerization. The QISA procedure according to this disclosure comprises the following steps: (1) preparing a gel that embeds individual cells or tissue pieces; (2) lysing the cells within the gel; (3) washing away the lysing reagents and substances of cellular or tissue origin, capable of inhibiting the subsequent amplification, under conditions causing DNA and/or RNA to precipitate; (4) drying the gel; (5) soaking the gel in a solution containing an enzymatic amplification system and a homogeneous fluorescent detection system; (6) incubating the gel under conditions providing for amplification of target RNA or DNA molecules; (7) monitoring the fluorescence produced by amplified targets; (8) estimating the local target concentration at a particular location within the gel by noting the moment when the target-specific fluorescence signal at that location grows substantially above the background level. If the purpose of an assay is just determining the presence or absence of a target in cells, either individual or present within the analyzed piece of a tissue, then step 8 may be omitted and target-specific fluorescence may be registered after completion of amplification.

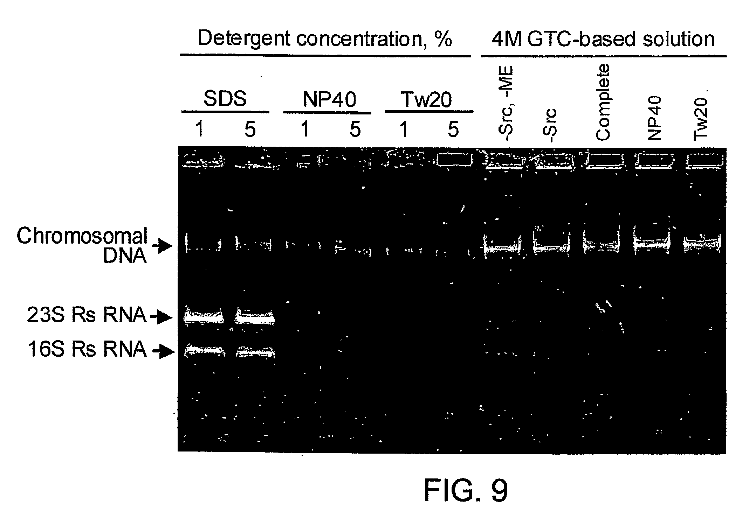

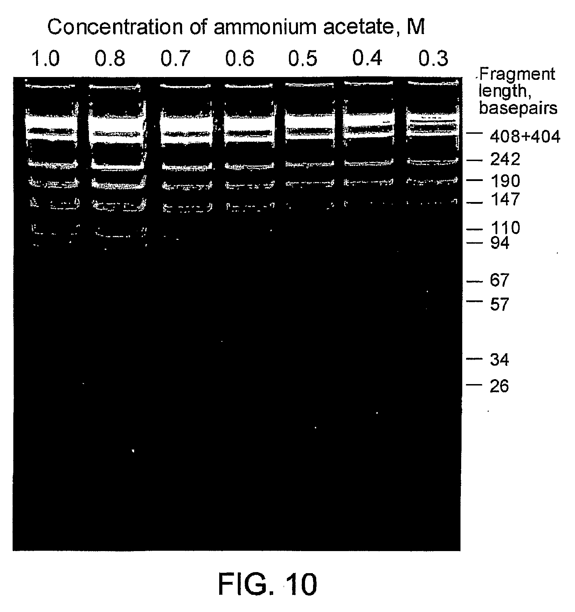

Following are comments on particular QISA steps.
(1) Embedding cells or tissue pieces can be achieved by including them into the polymerization mixture, together with monomer (for example, acrylamide), crosslinker (for example, N,N'-methylene-bisacrylamide) and polymerization catalysts (for example, a mixture of ammonium persulfate and N,N,N',N'-tetramethylethylenediamine). Preferably, embedded cells are arranged in a monolayer (or provided as a thin tissue slice) just beneath the gel surface. This ensures that nucleic acids of each cell are readily accessible to the enzyme(s) and other components of an amplification system (and hence that all targets will be amplified) and that all amplified targets are positioned in the same focal plane (and hence can be monitored simultaneously). This is especially important if the gel is much thicker than the cells or tissue slices to be analyzed. The desired arrangement of the analyzed material can be achieved in a number of ways, such as by flotation, including centrifugation-aided flotation; in this case, the polymerization solution should be made denser (heavier) than are the cells; alternatively, it may be made lighter than cells, and the cassette should be kept upside down during polymerization, to allow cells to sediment on a coverslip; and by the merged gels method disclosed in Example 9. In the latter case, polymerization is preferably carried out in a partially assembled chamber of this disclosure (Figure 7), whose one side is covered with a film of a first dry gel. After pouring the polymerization mixture into such a chamber, the first dry gel swells due to soaking in water and the low molecular weight constituents and displaces large particles towards the opposite side of the chamber, thereby functioning like fishing net. Upon polymerization, the second gel becomes merged with the first, whereas cells or tissue pieces are concentrated beneath the surface of the resulting gel. Chemically, the two gels can be either similar or different. For example, the first gel can be agarose, whereas the second gel can be polyacrylamide.
(2) Cells (or tissue sections) embedded within the gel can be lysed using any of known lysing solution which ensures a substantially complete lysis and substantial preservation of the integrity of nucleic acids to be assayed. Preferable lysis methods of this disclosure are those using solutions based on GTC (guanidine thiocyanate) or on SDS (sodium dodecyl sulfate), as disclosed in Example 10 and Figure 9 with E. coli cells as an example. Example 10 also provides methodology for checking if a given protocol is suitable for lysing a particular type of cells or tissue. The lysis solution may include lytic enzymes (along with, or instead of denaturants), such as lysozyme, a protease (for example, proteinase K), a lipase, etc. If only RNA or DNA is to be assayed, then the lysis solution may also include a deoxyribonuclease or a ribonuclease, respectively. If a protease and/or a nuclease is used for cell lysis, the gel should then undergo a treatment capable of inactivating the enzyme (for example, heating at 100°C, phenol extraction, or the addition of enzyme inhibitor).
(3) We discovered that nucleic acids embedded in a gel can be immobilized (lose the ability to diffuse) in the presence of agents and under conditions that cause precipitation of nucleic acids in solutions. Hence, solutions useful for precipitating nucleic acids and washing or extracting the resulting pellets are generally applicable to nucleic acids embedded in a gel. Accordingly, washing solution of this disclosure comprises a water-miscible organic solvent, capable of precipitating DNA and/or RNA. Organic solvents capable of precipitating nucleic acids are well known in the art; see, for example, U.S. Patent 5,945,515. Preferable solvents are lower alcohols, such as methanol, ethanol and isopropanol; however, other water miscible solvents indicated in the reference, as well as solvent mixtures, can be used. Preferably, washing solution also comprises a salt that aids precipitation due to shielding and hence weakening the repulsion between negative charges of the sugar-phosphate backbone of nucleic acids, and helps to remove proteins by weakening nucleic acid-protein interactions. (The repulsion can also be decreased by lowering pH value of the medium, due to protonation of the phosphate groups. However, this option is not recommended for the use with DNA targets since DNA is unstable in acidic media.) A variety of substances can be used as the salt constituent of a washing solution. We found that saline alcohol, obtained by mixing 45 volumes of 96% ethanol with 55 volumes of a solution containing 200 mM Na-citrate, 300 mM NaCl and 0.4 mM EDTA, efficiently extracts from a polyacrylamide gel a variety of low molecular weight substances and some proteins, yet preserves within the gel the DNA content of molecular colonies (Samatov et al., 2005). Various salts and combinations thereof can be used, such as chlorides, acetates and perchlorates of sodium, ammonium and lithium. In choosing a salt it is important to take into account that the salt should be readily soluble in a given mixture of organic solvent(s) and water, and it should not produce insoluble compounds with the components of a given lysing solution. For example, potassium salts should preferably be not used with lysing solutions containing dodecyl sulfate anions, because potassium dodecyl sulfate is hardly soluble at room temperature; however, potassium salts may be used with guanidinium-based lysing solutions. Optimal concentrations of organic solvent(s) and salt(s) in washing solution should be determined in preliminary experiments, such as the experiment described in Example 11 and Figure 10. Good washing solution should at least be a good solvent for the lysing reagents and, simultaneously, a poor solvent for nucleic acids. Further, it should preferably be capable of solubilizing at least some proteins and substances of cellular origin that can potentially inhibit the subsequent nucleic acid amplification. It is understood that if washing solution contains a high salt (that may be inhibitory for amplification), than the gel should be additionally washed with a solution comprising a salt-free (or low salt) organic solvent at a concentration that does not provide for solubilization of nucleic acids, such as 70% ethanol.
   A particular protocol may comprise consecutive washings of the gel with a series of washing solutions; the optimal scheme should be determined experimentally. Thus, the procedure of Example 12 includes consecutive washings with 70% isopropanol, then with a saline alcohol prepared by mixing 45 volumes of 96% ethanol with 55 volumes of 0.5 M ammonium acetate, and finally with 70% ethanol. This procedure is suitable for washing the gel from either a GTC- or an SDS-based lysing solution. Washing with saline alcohol is important when the analyzed sample contains large amounts of proteins, lipoproteins, glycoproteins, polysaccharides, and/or other substances (for example, hem) that may inhibit nucleic acid amplification.
   The optimal regimen of washing (time, temperature) depends on the composition of lysing solution and on the physical properties of the gel, such as thickness and density and should be determined experimentally. Removal from the gel of some constituents of a lysing solution can be monitored with specific reagents, and these constituents can serve as washing indicators. For example, residual thiocyanate anions can be detected through formation of a red-colored complex with Fe³⁺, whereas dodecyl sulfate anions can be detected through formation of a precipitate in the presence of K⁺. It is recommended that washing regimen used is several times more stringent than is required for reducing the amount of the indicator components below detectable level. Washing at ambient temperature is technically most simple, but complete removal of all components capable of inhibiting the subsequent amplification may require a considerable time. Duration of washing can be reduced by increasing temperature of the washing solution. We found that increasing washing temperature to 50°C substantially accelerates removal of the indicator components without appreciably affecting DNA pattern in the gel. Other temperatures can also be used. Preferably, washing temperature should be below the boiling temperature of the organic solvent (or its azeotropic point), to avoid a significant decreasing its concentration during washing.
(4) Drying of the gel should be substantially complete, because residual organic solvent can be inhibitory to subsequent amplification. Various drying methods may be used. The simplest method is to leave the gel on a bench at ambient temperature; for example, 30 min is enough to dry a 0.4 mm-thick polyacrylamide gel after the gel has been washed in 70% ethanol. During drying, the gel surface should be protected from dust and other contaminants.
   Steps 5 to 7 are identical to analogous steps in growing and monitoring molecular colonies produced by individual target molecules (see above), except that the soaking solution does not contain target molecules (but it may contain an internal standard, see below).
(8) To correctly estimate the local target concentrations, it is important to have some kind of a standard for the amount of target. As a standard, one can use a separate gel spotted with known amounts of target, like that shown in Figure 8. However, because reaction conditions in separate gels can be different, even in neighboring gels placed into the same PCR machine, it is preferable to use internal standards, i.e., those present in the same gel.

At least two types of internal standards can be used:
(a) Individual molecules of a template which possesses amplification properties very similar to those of the analyzed target, but which is hybridizable with a different fluorescent probe (preferable differently colored than the target-specific probe). Requirements for such an internal standard are essentially the same as those formulated for internal standards used in solution PCR (see, for example, Souazé et al., 1996; Willhauck et al., 1998; Freeman et al., 1999). It should be, however, noted that, in contradistinction to solution PCR, there exists substantially no competition between target and internal standard during in-gel amplification because they are spatially separated. Time (or cycle number) of the appearance of colonies produced by the internal standard molecules will, on one hand, indicate whether the reaction kinetics was as expected [for example, the colonies produced by individual molecules in the asymmetric PCR experiment of Example 4 (Figure 5, lower row of the top panel) appeared after cycle 33; a significant deviation by the same target from this number would indicate kinetic abnormalities] and, on the other hand, serve as a reference point helping to estimate the target concentrations at locations which become fluorescing earlier, taking into account that four PCR cycles translates into approximately one order of magnitude difference in the target copy number (see above and Figure 8).
(b) Calibration particles (for example, glass beads), each linked with a predetermined number of template strands. The particles are preferably arranged in a characteristic pattern (a code), for example, by gluing to a piece of a fine gauze, and are incorporated into the amplification gel. Because fluorescent spots produced by these particles are easily distinguishable by their characteristic pattern, the template linked to the particles may be the analyzed target itself, thus eliminating any errors that commonly result from differing amplification kinetics of target and internal standard. The pattern of calibrating particles can also serve for unambiguous orientation of the gel images. The template molecules can be linked to the particles either covalently or non-covalently. For example, it can be bound to the particles through hybridization with an immobilized oligonucleotide, or a biotin-modified template can be bound to immobilized streptavidin. Covalently linked template molecules are preferably tethered to the particles through a linker long enough to provide for a non-constrained template copying, or the template can be tethered through a cleavable linker which is cleaved before amplification (for example, during the lysis step). A number of cleavable linkers are known in the art, for example, linkers containing S-S-bond which can be broken by SH-compounds such as dithiothreitol or 2-mercaptoethanol (see, for example, Shirnkus et al., 1985), usually included into lysing solutions. After preparation of calibration particles, the exact number of template strands linked to a particle as well as the dispersion of a template-per-particle load can be ascertained using MCT, by determining the number of colonies produced by the strands released from individual particles.

QISA has important differences from *in situ* PCR. In conventional *in situ* PCR, cells are treated to make their walls semi-permeable, preferably permeable for PCR reaction components, including dNTPs, primers and DNA polymerase, but not permeable for template DNA or RNA and for the amplification products. Amplification is carried out in solution, in which the cells are suspended or which covers cells attached to the surface of a slide. Since it is not possible to simultaneously make the cell membrane readily permeable for a DNA polymerase and completely prevent any leakage of DNA, a certain (sometimes substantial) efflux of PCR products from the cells and their migration to other locations cannot be excluded. This may result in errors in the target localization and makes quantitative target assays hardly possible.

In the QISA method of the present disclosure, cells entrapped in a gel matrix are completely lysed. Therefore, there are no restrictions in the access of target molecules to the components of an amplification system. Yet, due to immobilization of the medium, target molecules and their copies remain at the locations formerly occupied by their home cells. The present disclosure utilizes for in-gel PCR a polyacrylamide or like gel that does not melt (does not transit to a sol) upon heating. Also, instead of using aqueous washing solutions in which nucleic acids are soluble, this disclosure teaches using solutions comprising water-miscible organic solvents capable of precipitating nucleic acids. Therefore, any RNA or DNA molecules contained in a cell retain their location, and their amplified copies also remain at that location. Finally, according to the present disclosure, amplified targets are preferably detected in real time, using a homogeneous fluorescent system included in the reaction cocktail. Hence, by noting the PCR cycle (or, in case of an isothermal amplification system, the time elapsed from the onset of the reaction) after which amplification products become visible at a certain location, it is possible to estimate the number of target DNA or RNA molecules in the cell (tissue area) embedded therein. Many cells can be analyzed in parallel in a single gel, for example, for the copy number of a certain mRNA. This would provide information on the level of expression of the corresponding gene in each of these cells, which may be useful for both research and diagnostic purposes.

For example, white cells isolated from the blood of a leukemia patient can be entrapped in a layer of polyacrylamide gel; the gel can then be treated to make the cells permeable or to bring about their complete lysis; the reagents used for cell lysis and the cell components which may be inhibitory for PCR are washed away under conditions that preserve the location of nucleic acids; then the gel is dried and processed using PCR primers and fluorescent probes specific to the mRNA associated with that particular leukemia type. The assay will show up (1) if there are malignant cells in the sample; (2) the proportion of the malignant cells to normal nucleated cells; and (3) the approximate copy number of the target RNA in each of the malignant cells.

The same approach can be used for the detection and quantitation of pathogenic microorganisms in clinical samples, such as in blood or in stool. A similar procedure can be used for the analysis of cancerous cells in bone marrow or in tissue slices (for example, in hepatoma biopsies) and for the determination of localization and local concentration of a certain mRNA species in tissues, organs, and embryos. After having analyzed a series of slices, it could be even possible to create a 3-D expression map of the corresponding gene in a tissue, an organ or even in an entire organism, such as in an embryo's body.

Furthermore, *in situ* assays according to this disclosure can be performed at sub-cellular level. The resolution power of the method can be increased by increasing the length of an amplified DNA. Also, molecular colonies can be made smaller by using denser gels, i.e., with a higher monomer concentration and a higher percentage of cross-links. It is even possible to make molecular colonies smaller than a eukaryotic cell whose diameter is usually larger than 10 µm (for example, Mitra et al, 1999 demonstrated molecular colonies as small as 6 µm), and hence to determine distribution and local density of a specific RNA species within the cell. For a sub-cellular analysis, the internal cell content (cytoplasm) should also be gelled, and this should be done with the native intracellular distribution of DNA and RNA molecules being preserved. There can be various means of achieving this goal. For example, the cell membranes can be made permeable for acrylamide, bisacrylamide and catalysts during the above disclosed embedment procedure (for example, by inclusion into the polymerizing solution detergents, water-miscible organic solvents, such as alcohols or dimethylsolfoxide, and also porins, proteases or lipases), so that polymerization monomers and catalysts will diffuse into cells without a substantial displacement of intracellular macromolecules. In contrast to conventional in situ PCR, in this case cell envelops need to be made permeable to low molecular weight compounds, and hence a very mild treatment is sufficient that does not make the cells leaky to proteins and nucleic acids. Alternatively, cells can be entrapped in an agarose gel or in a first polyacrylamide gel, which is then dried (preferably in a freeze-drier or after treatment with a fixative, such as alcohol, to maximally preserve the cell integrity) and soaked in a solution containing monomeric acrylamide and N,N'-methylene-bisacrylamide and polymerization catalyst(s). Partial drying (dehydration) of the gel may be sufficient, so that the gel becomes able to soak in the solution containing the polymerization monomers and catalysts. After drying, the cell membrane may become leaky to the monomers and catalysts, and this can be ascertained by inclusion in the solution of a water-soluble dye and checking whether the dye enter the cells. If needed, the membrane permeability can be increased as disclosed above. Whatever method of permeabilization of cells is used, it is preferable to use catalysts that can be triggered at a desired timepoint, such as a combination of TEMED and riboflavin, which can be activated by illumination; alternatively, premature polymerization can be prevented by cooling the gel. After equilibrating the monomers across the cell membranes (within several minutes, which can be checked by equilibration of a water-soluble dye), acrylamide polymerization can be induced by illuminating the sample or by raising the gel temperature.

For a number of diagnostic purposes, QISA can be an efficient alternative of amplification of individual target molecules, even if *in situ* amplification as such has no diagnostic value. In case of QISA, the analyzed sample needs no preliminary treatment, for example, isolation of nucleic acids which typically includes a number of manual operations, such as transfers, centrifugations and/or filtrations. Nucleic acids are isolated *in situ* by means of mere incubations and changing solutions, i.e., the procedures that are readily amenable for automation. Speed of the procedure can be significantly increased by rising the incubation temperatures and by using thinner gels. Thus, increasing temperature from 25 to 50°C can potentially reduce the time 10 to 20-fold, and decreasing gel thickness from 400 to 40 µm can result in a further 10-fold reduction. Therefore, using the same technology as disclosed in Example 12, the entire procedure of sample preparation can be made some 100 to 200 times faster than in that example, i.e., it will take minutes instead of about 20 h.

It should be noted that the product of the QISA procedure can be useful for a variety of purposes not related to homogeneous detection, such as for *in situ* sequencing (Mitra et al., 2003; Shendure et al., 2005) or *in situ* expression of certain genes (Samatov et al., 2005), or just for *in situ* nucleic acid isolation. For these applications, the inclusion of a homogeneous detection system in the soaking solution at step (5) is not necessary.

The first step of the QISA procedure (embedding cells in a gel) can also be used for preparing 2-D arrays of intact living cells, including cell arrays of a very high density. Preferably, embedded cells in a 2-D array are arranged in a monolayer, but for some applications (for example, for screening cell populations in which the sought cells are rare) cell layers whose thickness exceeds one cell diameter can be useful. Again, the 2-D arrangement of the cells can be achieved in a number of ways (see above), but we prefer the merged gels method according to this disclosure. The procedure for preparing preferred cell arrays with the merged gel method is generally the method disclosed in Example 9, except the preferred amount of cells in the injected suspension is high enough to provide for up to a maximally tight packing of the cells in a monolayer alongside the viewing window of the reaction chamber 7 (Figure 1). In addition to cells, the merged gels method can be used for arraying a variety of microscopic particles, such as nanoparticles, subcellular organelles (such as mitochondria and chloroplasts), ribosomes, paramagnetic beads, and even droplets of a double (water-in-oil-in-water) emulsion (Bernath et al., 2004). As in QISA, the merged gels can be either similar or different chemically. As far as preservation of the cell intactness is concerned, it may be preferred that the first (dried) gel is polyacrylamide, and the second gel is a low gelling temperature agarose, like one used for preparing Qβ replicase-containing gels (see Example 1 in U.S. Patents 5,616,478, 5,958,698 and 6,001,568, and in Russian Federation patents 2,048,522, 2,114,175 and 2,114,915).

There can be a huge amount of cells arrayed as a monolayer at the gel surface. Assuming that the diameter of a prokaryotic cell is ≈1 µm (as that of an average *Escherichia coli* cell), and the diameter of a eukaryotic cell is ≈10 µm (as that of an average human leukocyte), up to 150 million prokaryotic cells and up to 1.5 million eukaryotic cells can be arrayed as a monolayer at the surface of our standard gel (14 mm diameter, ≈1.5 cm² square). If, instead, the gel has the shape and size of a DVD (or CD) disk (≈100 cm² square), then it can accommodate up to 10¹⁰ prokaryotic cells (put another way, such a gel has a 10 Gigacell capacity, which is comparable to the standard DVD capacity, 4.5 Gigabyte) and up to 10⁸ eukaryotic cells (a 100 Megacell capacity). This provides for an unprecedented performance of the analysis (screening) of individual intact cells (as well as for the QISA procedure in which the embedded cells are lysed and the cellular nucleic acids are isolated *in situ*). For screening, the arrayed cells can be fluorescently labeled (for example, by a fluorescent antibody that binds with a 'receptor' protein on the cell surface, by a fluorescent protein that is synthesized within the cell, or by any of the various homogeneous fluorescent reporter systems discussed in this application that binds with a nucleic acid within the cell, such as by a molecular beacon probe).

At present, there are three basic technologies used for high-throughput cell screening: (1) presentation of cells by growing them as colonies on the surface of a nutrient gel (agar) (similarly, viruses are presented as viral plaques, cell-free lysis zones formed in a continuous lawn of host cells), (2) flow cytometry, and (3) laser scanning cytometry:
(1) Growing cell colonies or viral plaques are arranged in a 2-D pattern, but the capacity is some million times lower than cell arrays according to this disclosure due to their relatively large size, less than 10,000 individual bacterial colonies or viral plaques can be resolved on a Petri dish of a diameter similar to the diameter of a DVD disk (≈12 cm).
(2) Flow cytometry produces a 1-D presentation of cells streaming within a thin capillary (see, for example, Bonetta, 2005). Flow cytometry is also used for cell sorting (for example, cells bearing fluorescent protein or reporter are collected in a tube, whereas unlabeled or weakly labeled cells are discarded). Flow cytometry provides for high rates of screening (up to 100,000 cells pre second). However, the cells are not fixed, and the investigator cannot return to, visually inspect and interrogate specific cells having certain genetic, biochemical, or morphological properties; or to re-inspect cells after treating or retreating them with reagents or drugs. Also, the very high rates of the flow through the capillary may result in cell damage due to fluid pressures and shear forces. Moreover, even at the maximally achievable acquisition rate (100,000 events per second), a flow cytometer would require 100,000 seconds (28 hours) to analyze 10¹⁰ bacterial cells.
(3) Laser scanning cytometry (LSC, developed by CompuCyte Corporation, Cambridge, MA) can analyze individual cells arranged in 2-D patterns, including adherent cultured cells, tissue sections, cancer tissue imprints, and cytology smears, preserving the sample along with the exact position of each measured sample. However, at present, LSC cannot achieve the screening rates characteristic of flow cytometry because of the lack of efficient ways to prepare cell monolayers of sufficiently high density.

For the screening of intact cells according to this disclosure, it is preferable that the gel-containing chamber has the shape and size of a DVD, for having cell arrays be compatible with the existing DVD technology. A DVD drive can be modified to become a laser fluorescent scanner, preferably equipped with a confocal optics. One surface of the chamber would accommodate the gel, whereas the opposite surface would function like a formatted DVD. This would provide for recording the exact location of each individual cell, and would make it possible to precisely return to that cell later at any time - like the heads of a DVD drive find any particular byte of any file saved on the disk. Time required for screening the arrayed cells should be no greater than the time required to read a DVD, some 10 to 20 min, i.e., ≈100 times faster than screening of 10¹⁰ cells by a flow cytometer.

The 2-D arrangement provides for easy manipulation of embedded cells. To allow the cells to breath, the viewing side of the chamber is preferably made of a material permeable for gases, such as oxygen and carbon dioxide (or is replaced with a sheet made of such a material after the gel has formed). The composition of the liquid phase of the gel, the gaseous phase surrounding the chamber, and the temperature can be controlled to either allow or not allow the embedded cells to divide. Also, various agents can be introduced into, or removed from the gel by soaking, and the effects of these manipulations can be registered. The agents can be applied either evenly across the gel surface, or in a gradient, to study the concentration effects. The arrayed cells could be inoculated with desired substances, such as by using micromanipulator needles or liposomes, or transformed with plasmids or viruses.

If desired, after their inspection, intact living cells can be lysed to release high molecular weight components. For example, nucleic acids can be isolated *in situ* as disclosed herein. If desired, the nucleic acids can be further amplified *in situ* (such as by means of PCR or another primer-dependent amplification system), and their amount within cells can be assessed with the QISA procedure. In particular, this approach can be useful for diagnostics, for example, for detection and analysis of rare malignant cells among a huge amount of healthy cells, or for the detection and identification of rare pathogenic microorganisms in complex bacterial mixtures, such as isolated from a stool specimen.

High density cell arrays according to this disclosure can be also used for *in situ* sequencing of nucleic acids. For example, DNA fragments to be sequenced can be ligated into a vector (for example, a single-stranded DNA phage, such as M13, or a plasmid) and amplified within arrayed *E. coli* cells (with or without *in situ* cell multiplication). After *in situ* isolation of nucleic acids, the entire or partial sequence of the ligated fragments is determined by a known *in situ* sequencing protocol, such as by using cycles of sequencing by nucleotide addition (Mitra et al., 2003) or by ligation (Shendure et al., 2005). To prevent diffusion of the sequencing primer which is being extended, the primer can be immobilized on the gel matrix using, for example, the Acrydite™ chemistry (U.S. Patent 5,932,711). Using the high density cell arrays, up to 10¹⁰ different fragments can be sequenced simultaneously. In effect, this could provide for the sequencing of the entire human or like genome on a single DVD-sized gel.

### EXAMPLES

### Example 1: Preparing the amplification gels

For experiments reported in this application, we used the apparatus depicted in Figure 1. Polyacrylamide gels were cast in wells 3 (14 mm in diameter, 0.4 mm deep) drilled in 1 mm-thick glass microscopic slides 2 (Fisherfinest™ premium microscopic slides, plain; Cat. No. 12-544-4; Fisher Scientific, Pittsburgh, PA) at the workshop of the Institute of Protein Research (Pushchino, Moscow Region, Russia). Defatted by overnight soaking in 0.5 M NaOH and thoroughly washed slides were dried, and the inner surface of wells 3 was treated by PlusOne™ Bind-Silane A174 (Cat. No. 17-1330-01; Amersham Biosciences Trading GmbH, now part of GE Healthcare, Vienna, Austria) by applying to each well 80 µl of a diluted reagent (0.4% of the Silane in an aqueous solution of 2% acetic acid and 80% ethanol). After passive evaporation of the solvent (about 1 hour at room temperature), slides 2 were rinsed with water, washed in 20 ml/slide of ethanol by a 15-min shaking, rinsed again, and dried. Into each well 3, we poured 70 µl of a degassed solution containing 7% acrylamide (puriss; Cat. No. 01699; Fluka Chemie AG, Buchs, Switzerland), 0.07% UltraPure™ N,N'-methylene-bisacrylamide (Cat. No. 15516; GibcoBRL/Life Technologies Inc., now part of Invitrogen Corp., Frederick, MD) and 0.04% PlusOne™ ammonium persulfate (Cat. No. 17-1311-01; Amersham Biosciences), mixed immediately before use with N,N,N',N'-tetramethylethylenediamine (TEMED; Reanal, Budapest, Hungary) to the final concentration of 0.5%. During filling in, wells 3 were covered by sliding-over with another, well-free, microscopic slide (not shown) that was pre-treated with PlusOne™ Repel-Silane ES (Cat. No. 17-1330-01; Amersham Biosciences) according to manufacturer's instructions. After incubation at room temperature during 40 min and then at 4°C during 1 hour, the upper slide was removed, lower (gel-containing) slides 2 were boiled 3 × 20 min in 40 ml/slide of deionized water (Milli-Q^{®}; Millipore GmbH, Moscow, Russia) and dried overnight at room temperature. The dry gels retain their properties after having been stored in a closed container at the room temperature during at least 1.5 months.

Before experiments, the gels were reconstituted to their original volume by soaking in an amplification reaction mixture ("PCR cocktail"). To this end, 2 - 3 µl of mineral oil (for IR-spectroscopy; Cat. No. 69808; Fluka Chemie) were applied as a ring 4 around each well, the well was half-covered with a coverslip 5 (Menzel-Glaser® #1; Menzel GmbH + Co KG, Braunschweig, Germany) and the specified PCR cocktail (60 to 65 µl, to completely fill in the well without allowing air bubbles) was poured under the coverslip, with a simultaneous sliding of cover slip 5 over well 3 until the well was fully covered. The covered well was sealed with a piece of adhesive PCR Foil 6 (Cat. No. 0030 127.471; Eppendorf AG, Hamburg, Germany) in which a round window of a diameter equal to that of the well was cut out beforehand to create closed reaction chamber 7 (Figure 1), and incubated for 1.5 hours at 4°C or otherwise (for example, for 30 min at 60°C) prior to PCR, during which time the gel swelled and absorbed all the liquid. At this point chamber 7 contained immobilized medium.

### Example 2: In-gel PCR in the presence of intercalating dye

The PCR cocktail included in the reaction chamber (Figure 1) contained 50 mM Tris-HCl (pH 8.7) (Ultrapure; Cat. No. US75825; Amersham Biosciences), 2.5 mM MgCl₂ (AnalaR^{®}; Cat. No. 10149; BDH Ltd., Poole, England), 0.2 mM each of dATP, dGTP, dTTP and dCTP (Sequence Grade; Amersham Biosciences), 0.2 µM each of primer YgfpF [5'-GGATCCGTTCAACTAGCAGACCA-3' (SEQ ID NO: 1)] and primer YgfpR [5'-AATGATGATGATGATGATGAGAACC-3' (SEQ ID NO: 2)] (synthesized at ZAO Syntol, Moscow, Russia, http://www.syntol.ru), 1 mg/ml bovine serum albumin (BSA) (fraction V; Cat. No. 735094; Roche Diagnostics GmbH, Mannheim, Germany), 3.6 ng/µl His-tagged Taq DNA polymerase (prepared as described by Chetverina et al., 2002), approx. 30 molecules of DNA template [plasmid carrying a sequence encoding the Cycle 3 mutant of green fluorescent protein (GFP) (Crameri et al., 1996), obtained by inserting the GFP sequence, PCR-amplified using plasmid pBAD-GFPuv (Cat. No. 6078-1, Clontech Laboratories, Inc., Palo Alto, CA), a forward primer containing site BspHI and a reverse primer containing site SmaI, between sites NcoI and SmaI of plasmid pIVEX 1.3 WG (Cat. No. 3 728 803, Roche Diagnostics)], and 1 × SYBR Green I (a 1:10,000 diluted stock solution; Cat. No. S-7580; Molecular Probes, Inc., Eugene, OR). The gel was sealed and allowed to swell to create the immobilized medium as reported in Example 1. In this example target nucleic acid was added to the amplification medium prior to its immobilization in the gel matrix but after formation of the pre-dried gel. PCR amplification to create colonies of amplified product by MCT was performed by thermal cycling the immobilized medium containing the nucleic acid (here DNA) template molecules dispersed in the thin layer of immobilized medium. In this example, as in all other examples employing thermal cycling, we used a UNO-Thermoblock™ thermocycler (Cat. No. 050-300, Biometra biomedizinische Analytik Gmbh, Göttingen, Germany) equipped with a flatbed *"in situ"* module (Cat. No. 050-203). For this example the first three PCR cycles comprised melting at 94°C for 15 s, annealing at 55°C for 10 s, and extension at 72°C for 40 s, whereas in the subsequent cycles, the melting step was reduced to 6 s. The PCR product was 229 bp-long, and had the following sequence defined by the primers:

During amplification the sample fluorescence was monitored, in this and subsequent examples, as follows. After a specified cycle number the slide containing amplification gels was taken out from the PCR machine after the extension step, scanned at a 50-µm resolution at room temperature (≈23°C) with confocal microarray reader ScanArray Express (Cat. No. ASCEX01; PerkinElmer, Inc., Downers Grove, IL), using blue (488 nm) laser to excite fluorescence and appropriate filters to register the desired emission light, after which PCR was continued, the next cycle beginning with the melting step. Figures reporting experimental results identify the PCR cycles after which readings were taken.

In this example, the SYBR Green I fluorescence was monitored using a 522-nm filter. Results are presented in Figure 2, which presents fluorescence images recorded using the microarray reader.

In this and subsequent examples, the specified number of plasmid molecules in a sample was obtained by serially diluting with 0.1% PEG 6000 (for synthesis; Art. 807491; Merck, Schuchardt, Germany) and 10 ng/µl polyadenylic acid (Cat. No. P 9403; Sigma Chemical Company, St. Louis, MO] in buffer TE [10 mM Tris-HCl (pH 9.0), 1 mM EDTA (ethylenediamine tetraacetate; analytical grade, Cat. No. 11280, Serva Feinbiochemika, Heidelberg, Germany)], of a stock solution of a plasmid whose concentration was determined spectrophotometrically assuming that 1 OD₂₆₀ unit equals 50 µg/ml of a double-stranded DNA (Appendix 8 to Sambrook et al., 2001), and taking into account the plasmid size and the Avogadro's Number (6.02 ×10²³) for converting the measured DNA amount into the number of molecules.

Referring to Figure 2, it is seen that the brightness of the immobilized medium increased generally with the number of thermal cycles. However, there is no indication of growing molecular colonies from individual templates. We believe that the dark spots in the images of Figure 2 and other figures are droplets of mineral oil from construction of the reaction chamber.

### Example 3: Estimating the sensitivity of homogeneous fluorescent detection systems

### (a) Detection of a double-stranded DNA using adjacently hybridizing FRET probes

A dry gel prepared as in Example 1 was soaked with a solution mimicking a PCR cocktail [50 mM Tris-HCl (pH 8.7), 2.5 mM MgCl₂, and 1 mg/ml BSA], dried in vacuo using freeze drier Hetosicc CD52 (Heto Lab Equipment A/S, Birkerod, Denmark). The gel was next spotted with five 0.5-µl aliquots containing known amounts (10⁶,10⁷, 10⁸, 10⁹, and 10¹⁰ molecules) of the PvuI-digested pC3GFP double-stranded DNA template (target) in a pattern shown in Figure 3 (top row). After the spots dried (within 15 min at room temperature), the gel was again soaked in a solution containing, in 1× TE buffer (see above), 0.1 µM each of Y donor probe [5'-GGGCAGATTGTGTCGACAGGTAATGGT-3'-FAM (SEQ ID NO: 4), complementary to the region of the target sequence (SEQ ID NO: 3) underlined with a double line] and Y acceptor probe [Cy5-5'-TCTGGTAAAAGGACAGGGCCATCGCCA-3'-PO₄ (SEQ ID NO: 5), complementary to the region of the target sequence (SEQ ID NO: 3) underlined with a single line], synthesized at ZAO Syntol, wherein FAM is carboxyfluorescein and Cy5™ is a cyanine dye (trademark of Amersham Biosciences); the fluorophores were tethered to the oligonucleotides through a PO₄(CH₂)₆NH₂ linker (http://www.syntol.ru). Upon hybridization with the target sequences, the two probes were spaced by two nucleotides (see above, SEQ ID NO: 3). The gel was sealed (see Example 1) and, after swelling during 1 hour at 4°C, it was subjected to "melting" (1 min at 94°C) and "annealing" (1 min at 52°C) in the PCR machine described in Example 2, and scanned at 670 nm (emission maximum of the Cy5™ fluorophore) using the conditions and the microarray reader also described in Example 2. The result is shown in the top panel of Figure 3.

It is seen that there is no evidence of the spotted amounts of even 10¹⁰ target molecules.

### (b) Detection of a single-stranded RNA using adjacently hybridizing FRET probes

The immobilized medium was prepared as described in Example 3(a), except that gel was spotted with aliquots containing known amounts (10⁷, 10⁸, 10⁹, 10¹⁰, and 10¹¹ molecules) of YGFP mRNA single-stranded target prepared by transcription of the PvuI-digested pC3GFP using T7 RNA polymerase (Gurevich et al., 1991) in a pattern shown in Figure 3 (bottom row). The immobilized medium containing the probes and targets was scanned at 670 nm after swelling (60 min at 4°C), then again after heating for 10 min at 60°C, and finally again after heating for 1 min at 94°C followed by 1 min at 52°C. The concentration of RNA for spotting the gel was determined spectrophotometrically assuming that 1 OD₂₆₀ unit equals 38 µg/ml (Appendix 8 to Sambrook et al., 2001). Results are shown in the bottom panel of Figure 3. It is seen that no spotted amounts of the mRNA target were detected when there was no annealing step, but that with both annealing procedures the spotted amounts of 10¹⁰ target molecules and 10¹¹ target mRNA molecules were detected.

### (c) Detection of in situ transcribed DNA using adjacently hybridizing FRET probes

A gel was spotted with a double-stranded DNA target as in Example 3(a), and then soaked in 65 µl of a solution containing, in addition to 0.1 µM each of the Y donor (SEQ ID NO: 4) and Y acceptor (SEQ ID NO: 5) probes, 100 mM Tris-HCl (pH 8.0), 20 mM MgCl₂, 1 mM spermidine (Cat. 100472, ICN Biomedicals, Inc., Irvine, CA), 0.2 mM EDTA, 40 mM UltraPure™ dithiothreitol (enzyme grade, Cat. No. 15508-013, GibcoBRL/Life Technologies Inc.), 4 mM each of ATP (Cat. No. 127531, Roche Diagnostics GmbH), GTP (Cat. No. 51120, Fluka Chemie), CTP (Cat. No. 30320, Fluka Chemie) and UTP (Cat. No. 101217, ICN Biomedicals, Inc., Irvine, CA), and 50 ng/µl T7 RNA polymerase isolated as described (Davanloo et al., 1984). The gel was sealed and scanned at 670 nm after swelling during 60 min at 4°C, then after 60-min and 120-min incubation at 37°C, and finally after heating for 1 min at 94°C followed by 1 min at 52°C. The result is shown in the top panel of Figure 4. It is seen that, when the immobilized medium was first incubated at 37°C for 2 hr for transcription followed by an annealing procedure, the spots to which were added 10⁸ DNA molecules, 10⁹ DNA molecules and 10¹⁰ DNA molecules were detected.

### (d) Detection of in situ transcribed DNA using a molecular beacon probe

Experiment was carried out as described in Example 3(c), except that instead of Y donor and Y acceptor probes, the soaking solution contained 0.1 µM molecular beacon Y [FAM-5'-CCGCGTCCATGCCATGTGTAATCCCAGCAGCGCGG-3'-BHQ-1 (SEQ ID NO: 6), complementary to the region of the target sequence (SEQ ID NO: 3) underlined with a dotted line, synthesized at ZAO Syntol, wherein BHQ is Black Hole Quencher™ (trademark of Biosearch Technologies, Novato, CA)], and the gel was scanned at 508 nm. The probe had a double-stranded stem five basepairs long (not complementary to the target) and a single-stranded loop 25 nucleotides long that was complementary to the target. The result is shown in the bottom panel of Figure 4. It is seen that none of the spots was detected. Because this example was only a demonstration, no attempt was made to adjust the probe, probe concentration, or the annealing conditions.

### Example 4: Non-invasive detection of molecular colonies in a sealed gel, using adjacently hybridizing FRET probes

The template DNA used in this example was a plasmid (kindly provided by M. V. Falaleeva, Institute of Protein Research) carrying a fragment of cDNA of chimeric AML1-ETO mRNA associated with the acute myeloid leukemia caused by chromosomal translocation t(8;21)(q22;q22) (Viehmann et al., 2003) involving genes AML1 (GeneBank accession number D43969) and ETO (GenBank accession number D14289). The plasmid was prepared as follows. A region of the AML1-ETO mRNA was amplified by RT-PCR using the total RNA preparation, isolated from leukocytes of a patient suffering from this type of leukemia, and 5'-phosphorylated primers AML1-A [5'-CTACCGCAGCCATGAAGAACC-3' (SEQ ID NO: 7)] and ETO-B [5'-AGAGGAAGGCCCATTGCTGAA-3' (SEQ ID NO: 8)] (the primers were as described by van Dongen et al., 1999). The 395-bp amplification product was ligated into a vector, pTZ19R (GeneBank accession number L08957), that had been digested with restriction endonuclease SmaI and dephosphorylated. The cloned insert was verified by sequencing. The resulting sequence, had a single base substitution compared with the published one (Miyoshi et al., 1993): t → c, just downstream of lowercase aa, the fused nucleotides of the AML1 and ETO genes.

The procedure of Example 2 was followed for the preparation of an immobilized medium. However, the template, primers and homogeneous fluorescence detection system in the PCR cocktail were different. The primers used in this example were as recommended by the European BIOMED-1 Concerted Action 'Investigation of minimal residual disease in acute leukemia: international standardization and clinical evaluation' for the detection of minimal residual disease with this type of chromosomal aberration (van Dongen et al., 1999). The forward primer, AML1-C [5'-ATGACCTCAGGTTTGTCGGTCG-3' (SEQ ID NO: 10)] was present at a concentration of 0.2 µM, and reverse primer, ETO-D [5'-TGAACTGGTTCTTGGAGCTCCT-3' (SEQ ID NO: 11)], was present at a concentration of either 0.2 µM (for symmetric PCR: Figure 5, upper row of the top panel) or 0.06 µM (for asymmetric PCR: Figure 5, lower row of the top panel). The priming regions are shown in bold in the above fused gene sequence (SEQ ID NO: 9), and expected length of the PCR product, as determined by the primers, is 260 bp.

The probes used in this example were as follows: L donor [5'-TGGCATTGTGGAGTGCTTCTCAGTACG-3'-FAM (SEQ ID NO: 12)], complementary to the double-underlined portion of the ETO sequence (downstream of the fusion site, see above sequence, SEQ ID NO: 9) and L acceptor [Cy5-5'-TTCGAGGTTCTCGGGGCCCATC-3'-PO₄ (SEQ ID NO: 13)], complementary to the single-underlined portion of the AML1 sequence (upstream of the fusion site). Upon hybridization with the chimeric target sequences, the two probes were spaced by two nucleotides (see above, SEQ ID NO: 9). The probes each were added at a concentration of 0.10 µM.

The primers and probes were synthesized at ZAO Syntol as described above (Examples 2 and 3). After soaking with the PCR cocktail and sealing (Example 1), the gel was subjected to PCR cycles as in Example 2, except that the extension steps lasted longer (80 s). After specified cycle numbers, noted in Figure 5, the amplification gels were scanned using blue (488 nm) laser to excite the FAM groups and a 670-nm filter to monitor the Cy5 fluorescence (Figure 5, top panel). It is seen that both symmetric PCR amplification and asymmetric PCR amplification generated colonies of amplified product that became visible after about 30 cycles of amplification. Colonies from symmetric PCR became visible a few cycles earlier, but colonies from asymmetric PCR became noticeably brighter beginning at cycle 35.

### Example 5: Non-invasive detection of molecular colonies in a sealed gel, using a molecular beacon probe

The experiment was carried out as in Example 2, except that the PCR cocktail contained 0.02 ng/µl His₆-tagged Pwo DNA polymerase (Dabrowski et al., 1998) in addition to Taq DNA polymerase and 0.03 µM molecular beacon Y (SEQ ID NO: 6) instead of the SYBR Green I dye, the concentration of primer YgfpR was 0.04 µM, and the gel was scanned at 508 nm after the specified PCR cycles (Figure 5, middle panel).

It is seen that, although the concentration of molecular beacon Y was 30% of the concentration of the same probe used in Example 3(d), colonies of amplified product became visible after about 30 cycles of amplification, with a brightness intermediate the brightnesses of the upper and lower rows of the top panel.

### Example 6: Non-invasive detection of molecular colonies in a sealed gel, using FRET between an intercalating dye and a hybridized fluorescent probe

The experiment was carried out as in Example 2, except that the PCR cocktail contained 0.1 µM Y acceptor probe (SEQ ID NO: 5) in addition to the SYBR Green I dye, the concentration of primer YgfpR (SEQ ID NO: 2) was 0.04 µM, and the gel was scanned after the specified PCR cycles (Figure 5, bottom panel) at 670 nm (upper row; to monitor the fluorescence of Cy5 residing at the Yacceptor probe, due to FRET from SYBR Green I) and at 522 nm (lower row; to monitor the fluorescence of SYBR Green I, not mediated by FRET).

It is seen that colonies of amplified product became highly visible at the wavelength of the probe by cycle 38. It is seen also that colonies of amplified product became visible at the wavelength of the nucleic acid binding dye a few cycles earlier, but that the brightness at the wavelength did not increase as much with additional cycles. The significant difference between Example 2, in which colonies were not detected at the wavelength of the dye, and this example, in which colonies were detected, was the presence of the Y acceptor probe.

### Example 7: Measuring the fluorescence emitted by an individual molecular colony

The fluorescence signal produced by an individual DNA colony in Figure 5, top panel, bottom row was analyzed to obtain a curve of fluorescence versus PCR cycle number. The signal in 16-bit TIFF images scanned after the specified PCR cycles was measured using the OptiQuant™ Image Analysis Software (Packard Instrument Company, Meriden, CT; now part of PerkinElmer, Inc.) and expressed as DLU (Digital Light Units as defined in OptiQuant™ Image Analysis Software Operation Manual, Publication Number 1694156 Rev. D, Packard Instrument Company, 1998). A "DLU" versus "PCR cycle number" plot built for one colony is shown in Figure 6. It will be appreciated that the time course of fluorescence from an individual colony resembles the time course of fluorescence of solution PCR beginning with few templates (Wittwer et al., 1997a).

### Example 8: Non-invasive monitoring of in-gel amplification of varying amounts of DNA

A dry gel disclosed in Example 1 was spotted with four 0.2-µl aliquots containing known amounts (1, 10, 100, and 1000 molecules) of the PvuI-digested pC3GFP (See Example 2, SEQ ID NO: 3), prepared by serially diluting the plasmid's stock solution in the dilution buffer of Example 2. After the spots had dried, the gel was soaked with the PCR cocktail and processed as in Example 2, except that instead of dye SYBR Green I, said cocktail contained 0.1 µM each of Y donor (SEQ ID NO: 4) and Y acceptor probe (SEQ ID NO: 5) disclosed in Example 3(a), the PCR cocktail contained no template, and the concentration of primer YgfpR was 0.04 µM. After the indicated number of PCR cycles, the gel was scanned at 670 nm. The results are presented in Figure 8. It is seen that spots containing more target molecules at the start of amplification became visible earlier. Spots containing 1000, 100, 10 and one target molecule became visible after cycles 26, 30, 34 and 36, respectively.

### Example 9: Merging gels method for arranging cells in a monolayer beneath gel surface

The first (bottom) gel was prepared essentially as described in Example 1, with the following modifications. Concentrations of the monomer and the crosslinker were lower: the concentration of acrylamide was 4% (concentrations of 3 to 5% were found to be satisfactory), whereas the concentration of N,N'-methylene-bisacrylamide was 1/100th of that of acrylamide. After incubation at room temperature during 40 min and then at 4°C during 1 hour, the first gel was dried overnight without washing. The second gel was cast with embedded cells by overlaying the first dry gel with a mixture containing 6% acrylamide (concentrations of 3 to 8% also gave satisfactory results), N,N'-methylene-bisacrylamide (1/100th part of acrylamide), 0.04% ammonium persulfate, 0.5% TEMED, 0.9% NaCl, and *E. coli* B834(DE3)/pLysS cells transformed with plasmid pC3GFP (cf. Example 2), approx. 20 or 200 cells per gel. After incubation at room temperature for 40 min and then at 4°C for 1 hour, the merged gels were dried in a freeze drier for 30 min before subjecting to QISA procedure according to Example 12.

In this example illustrating the merged gel method, the first dry gel swells, by soaking in low molecular weight ingredients of the cell-containing polymerization solution, and fills essentially the entire inner volume of the reaction chamber, except the volume occupied by cells. The cells, which are too big to be soaked up by the first gel, are displaced to the coverslip that closes the chamber. After formation of the second gel, which polymerizes within the first gel and hence becomes almost entirely merged with the first gel, the cells are arranged in a layer just beneath the flat merged gel surface formed by the coverslip.

### Example 10: Testing cell lysis methods

*E. coli* cells were grown in 20 ml of L-broth at 37°C, in a shaker set to 150 rpm, during 1.5 h until optical density at 590 nm reached 0.7. Cells containing in a 1.5-ml aliquot of the culture (approx. 4 × 10⁸ cells) were pelleted in centrifuge 5414C (Eppendorf) during 10 min at 3000 rpm, washed by suspending in 1 ml of 0.9% NaCl, pelleted again, and subjected to one of the following lysis procedures to ascertain its suitability for lysing this type of cells, particularly for use in the QISA procedure.

### (a) Lysis with a detergent-based solution

The pellet was suspended in 500 µl of a solution containing 50 mM Tris-HCl (pH 7.5 at 20°C), 100 mM NaCl, 20 mM EDTA (pH 8.0 at 20°C), 2% 2-mercaptoethanol, and one of the following detergents: 1% or 5% SDS (lanes "SDS" in Figure 9), 1% or 5% Nonidet P40 (lanes "NP40" in Figure 9), or 1% or 5% Tween 20 (lanes "Tw20" in Figure 9). After incubation during 5 min at room temperature, cell debris was pelleted in centrifuge MiniSpin™ (Eppendorf) during 15 min at the maximum speed.

### (b) Lysis with a guanidinium-based solution

The pellet was suspended in 200 µl of either a "complete" lysis solution containing 4 M GTC (guanidine thiocyanate; Cat. No. 50990, Fluka Chemie), 25 mM Na-citrate (pH 7.0 at 20°C), 0.5% Sarcosyl™ NL30 (Cat. No. 44275, BDH Ltd., Poole, Dorset, UK), 13 mM EDTA (pH 8.0 at 20°C), and 1.3% 2-mercaptoethanol (lane "Complete" in Figure 9), or a solution in which Sarcosyl™ NL30 was omitted alone (lane "-Src" in Figure 9) or omitted together with 2-mercaptoethanol (lane "-Src, -ME" in Figure 9), or replaced with 1% Nonidet P40 (lane "NP40" in Figure 9) or with 1% Tween 20 (lane "Tw20" in Figure 9). After incubation for 10 min at room temperature with occasional vortexing, cell debris was pelleted in a MiniSpin™ centrifuge (Eppendorf) during 15 min at the maximum speed.

A clarified lysate obtained by each procedure was mixed with 2 volumes of isopropanol. After incubation at -20°C for 30 min, the precipitate that formed was pelleted in the MiniSpin™ centrifuge for 15 min at the maximum speed. The pellet was suspended, by vortexing for 10 min in a Reax-Top™ mixer (Heidolph Instruments GmbH & Co. KG, Schwabach, Germany) at the first speed (approx. 500 rpm), in 100 µl of a saline alcohol, obtained by mixing 45 volumes of 96% ethanol with 55 volumes of a solution containing 200 mM Na-citrate, 300 mM NaCl and 0.4 mM EDTA. After having been pelleted again and washed with 100 µl of 80% ethanol, a sample was dissolved in 500 µl of a solution containing 50 mM Tris-HCl (pH 7.5 at 20°C), 100 mM NaCl, 20 mM EDTA (pH 8.0 at 20°C), 2% 2-mercaptoethanol and 5% SDS. A 1/50th part of the so obtained solution was subjected to electrophoresis through a 1% agarose gel followed by staining with ethidium bromide (Sambrook et al., 2001). The stained electrophoresis gel is shown in Figure 9.

Bands for *E. coli* chromosomal DNA, 23S ribosomal RNA and 16S ribosomal RNA are identified by arrows in Figure 9. It is seen that all lanes have a significant band for chromosomal DNA, but the GTC-based solutions recovered more chromosomal DNA. It is also seen that both SDS lanes and all GTC lanes have distinct bands for both non-degraded ribosomal RNA's. For that reason we judged SDS-based and GTC-based lysing solutions to be preferred for this cell type.

### Example 11: Optimizing the composition of washing solution

This example demonstrates finding the optimal concentration of a salt at a fixed concentration of an organic solvent. Optimal concentration of an organic solvent at a fixed concentration of a salt can be found similarly. The optimal concentrations can be utilized in the QISA procedure.

Seven aliquots each containing 200 ng of plasmid pUC129, digested with restriction endonuclease HpaII, and 10 µg of a liquid (non-cross-linked) polyacrylamide (Gaillard et al., 1990) in 300 mM NaCl were mixed with 3 volumes of ethanol, incubated for an hour at -20°C, and centrifuged in a MiniSpin™ centrifuge (Eppendorf) at the maximum speed for 15 min at 4°C. Each DNA pellet was suspended in 100 µl of a saline alcohol obtained by mixing 45 volumes of 96% ethanol and 55 volumes of a solution of ammonium acetate whose concentration was 1.0, 0.8, 0.7, 0.6, 0.5, 0.4, or 0.3 M. Suspensions were vortexed during 15 min in a Reax-Top™ mixer (Heidolph Instruments GmbH & Co. KG, Schwabach, Germany) at the first speed (approx. 500 rpm) and again pelleted as above. Each pellet was washed in 100 µl of 80% ethanol, dried, dissolved in 10 µl of buffer TE (see Example 2), and analyzed by electrophoresis through a non-denaturing 8% polyacrylamide gel followed by staining with ethidium bromide (Sambrook et al., 2001). The stained electrophoresis gel shown in Figure 10 shows that as ammonium acetate concentration was reduced to 0.3M, preservation of the larger DNA fragments decreased slightly, and that as ammonium acetate concentration was increased to 1.0M, preservation of smaller DNA fragments increased. Concentration of 0.5 M was found to be optimal, since at this ammonium acetate concentration, DNA fragments of ≥200 bp (the usual size of amplified targets) are substantially preserved, whereas shorter fragments tend to be dissolved. The latter serves as an indicator of conditions that also provide for the dissolution of many proteins and PCR inhibitors often present in biological samples (our unpublished observations).

### Example 12: QISA procedure

Each dry gel of Example 9 (merged gels, containing embedded *E. coli* cells carrying plasmid pC3GFP) was overlaid with 50 µl of one of the following two lysing solutions: either (a) 4 M GTC (purum, Fluka AG, Switzerland), 25 mM Na-citrate (pH 7.0), 1 mM EDTA, 1.33% 2-mercaptoethanol or (b) 1% SDS, 10 mM Tris-HCl pH (7.5), 1 mM EDTA, 2% 2-mercaptoethanol. After a 10-min incubation at 25°C (a gel soaked in the entire lysis solution within 5 min) gels were washed as follows. A slide containing 3 gels was placed upside down in a glass Petri dish of 95 mm internal diameter, at a height of 5 mm above the dish bottom. A washing solution was then poured into the dish in an amount sufficient to cover the slide (40 - 45 ml). Washing was performed without agitation, taking advantage of natural convection, so that the denser lysing solution diffusing out from the gel fell down on the bottom of the dish without being mixed with a body of the washing solution, so that the gels were mostly contacting with fresh portions of the solution. However, this manner of washing is not mandatory; other variants will also give satisfactory results (for example, positioning the slides upside up and continuous or intermittent stirring the washing solution). Gels were washed at 25°C, consecutively (1) in 70% isopropanol for 30 min, (2) in a saline alcohol obtained by mixing 45 volumes of 96% ethanol and 55 volumes of 0.5 M ammonium acetate (cf. Example 11) for 1 h, (3) in three changes of 70% ethanol for 30 min, 2 h, and overnight. After the gels were dried at room temperature for 30 min, they were soaked with the PCR cocktail and processed as in Example 8, except that primer YF414 [5'-TCAAGTTTGAAGGTGATACCCTTGT-3' (SEQ ID NO: 14)] was used instead of YgfpF, resulting in a 414 bp-long amplification product.

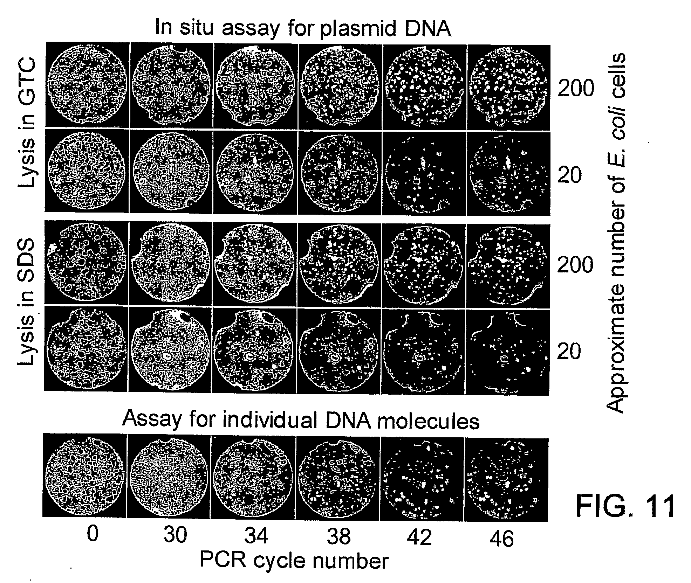

Results are shown in Figure 11. Two top rows show gels treated with a GTC-based lysing solution; the next two lower rows show gels treated with an SDS-based lysing solution, and the bottom row shows a control GTC-treated gel which contained no cells, but contained approx. 40 individual molecules of the PvuI-digested pC3GFP which were added to the PCR cocktail. It should be noted that the final density of the gels (10% acrylamide) is higher than of the gels of Figure 5 (7% acrylamide, cf. Example 1); therefore, the size of molecular colonies is smaller.

It is seen from Figure 11 that (1) the number of fluorescing spots correlated with the number of *E. coli* cells introduced into the gels during polymerization; (2) both the GTC- and SDS-based lysing solutions gave satisfactory results; (3) the GTC-based solution recovered more plasmid-containing cells, in agreement with the results of Figure 9 (cf. Example 10) showing that lysis with GTC gives a higher yield of DNA than does lysis with SDS, whereas lysis with SDS gives a higher yield of ribosomal RNA; (4) in gels containing *E. coli* cells, fluorescing spots tend to appear earlier than in the gel with individual plasmid molecules, indicating that cells may contain multiple copies of the plasmid; (5) the fluorescing spots corresponding to cells appear less synchronously than those corresponding to individual molecules, indicating that there may be a scatter in the plasmid copy number per cell.

In summary, the results of Figure 11 demonstrate that (1) cells embedded in a gel can be efficiently lysed with solutions developed for the lysis of suspended cells; (2) the washing scheme employed removes from the gel the lysing reagents and all substances of cellular origin that might interfere with PCR; (3) the released nucleic acid target, in this case DNA, remains immobilized during cell lysis, washing and PCR, despite there being no treatment with a fixing reagent (like formaldehyde) which is mandatory in the conventional *in situ* PCR; (4) the *in situ* amplified DNA can be detected without any post-amplification treatment and without opening the reaction chamber, which was not possible in the conventional *in situ* PCR; (5) the method of the disclosure provides for quantitative assays of the *in situ* amplified targets. In other words, the results of Figure 11 demonstrate the feasibility of QISA.

### REFERENCES

Afonia, I.A., et al., "Minor Groove Binder-Conjugated DNA Probes for Quantitative DNA Detection by Hybridization-Trigger Fluorescence," BioTechniques 32: 940-949 (2002).
Agrawal, S., Ed., "Protocols for Oligonucleotides and Analogs: Synthesis and Properties", Humana Press, Totowa, NJ (1993).
Bengtsson, M., et al., "A new minor groove binding asymmetric cyanine reporter dye for real-time PCR", Nucleic Acids Res., vol. 31, article e45 (2003).
Bernath, K., et al., "In vitro compartmentalization by double emulsions: sorting and gene enrichment by fluorescence activated cell sorting", Anal Biochem., vol. 325, pp. 151-157 (2004).
Bonetta, L. "Flow cytometry smaller and better", Nature Methods, vol. 2, pp. 785-795 (2005).
Butz, J., et al., "Characterization of mutations and loss of heterozygosity of p53 and K-ras2 in pancreatic cancer cell lines by immobilized polymerase chain reaction", BMC Biotechnology, vol. 3, article 11 (2003).
Cardullo, R.A, et al., "Detection of nucleic acid hybridization by nonradiative fluorescence resonance energy transfer", Proc. Natl. Acad. Sci. USA, vol. 85, pp. 8790-8794 (1988).
Chen, X., et al., "A homogeneous, ligase-mediated DNA diagnostic test", Genome Res., vol. 8, pp. 549-556 (1998).
Chetverin, A.B., et al., " Nonhomologous RNA recombination in a cell-free system: evidence for a transesterification mechanism guided by secondary structure", Cell, vol. 88, pp. 503-513 (1997).
Chetverin, A.B., et al., "On the nature of spontaneous RNA synthesis by Qβ replicase", J. Mol. Biol., vol. 222, pp. 3-9 (1991).
Chetverina, H.V., et al., "Cloning of RNA molecules in vitro", Nucleic Acids Res., vol. 21, pp. 2349-2053 (1993).
Chetverina, H.V., et al., "Molecular colony diagnostics: Detection and quantitation of viral nucleic acids by in-gel PCR", BioTechniques, vol. 33, pp. 150-156 (2002).
Compton, J., "Nucleic acid sequence-based amplification", Nature, vol. 350, pp. 91-92 (1991).
Crameri, A., et al., "Improved green fluorescent protein by molecular evolution using DNA shuffling", Nat. Biotechnol., vol. 14, pp. 315-331 (1996).
Dabrowski, S., et al. "Cloning and expression in Escherichia coli of the recombinant His-tagged DNA polymerases from Pyrococcus furiosus and Pyrococcus woesei", Protein Expr. Purif., vol. 14, pp. 131-138 (1998).
Dattagupta, N., et al., "Isothermal Strand Displacement Nucleic Acid Amplification," European Patent Application No. 0 676 476 (1995).
Davanloo, P., et al., "Cloning and expression of the gene for bacteriophage T7 RNA polymerase", Proc. Natl. Acad. Sci. USA, vol. 81, pp. 2035-2039 (1984).
Di Giusto, D.A., et al., "Proximity extension of circular DNA aptamers with real-time protein detection", Nucleic Acids Res., vol. 33, article e64 (2005).
Eckstein, F., Ed., "Oligonucleotides and Analogues: a Practical Approach", IRL Press, Oxford (1991).
Egholm, M., et al., "PNA hybridizes to complementary oligonucleotides obeying the Watson-Crick hydrogen-bonding rules", Nature, vol. 365, pp. 566-568 (1993).
Fire, A., et al., "Rolling replication of short DNA circles", Proc. Natl. Acad. Sci. USA, vol. 92, pp. 4641-4645 (1995).
Förster, T., "Zwischenmolekulare energiewanderung and fluoreszenz", Ann. Physik., vol. 2, pp. 55-75 (1948).
Freeman, W.M., et al., "Quantitative RT-PCR: pitfalls and potential", BioTechniques, vol. 26, pp. 112-122, 124-125 (1999).
Gaillard, C., et al., "Ethanol precipitation of DNA with linear polyacrylamide as carrier", Nucleic Acids Res., vol. 18, p. 378 (1990).
Guatelli, J.C., et al., "Isothermal, in vitro amplification of nucleic acids by a multienzyme reaction modeled after retroviral replication", Proc. Natl. Acad. Sci. USA, vol. 87, pp. 1874-1878 (1990).
Gurevich, V.V., et al., "Preparative in vitro mRNA synthesis using SP6 and T7 RNA polymerases", Anal. Biochem., vol. 195, pp. 207-213 (1991).
Haase, A.T., et al., "Amplification and detection of lentiviral DNA inside cells", Proc. Natl. Acad. Sci. USA, vol. 87, pp. 4971-4975 (1990).
Haugland, R.P., "Nucleic acid detections and genomics technology", in: "Handbook of Fluorescent Probes and Fluorescent Products", 9th Edn., Molecular Probes, Inc., Chapter 8 (2002).
Heller, M.J., et al., "Homogeneous nucleic acid hybridization diagnostics by nonradiative energy transfer", European patent application EP0070685 (1983).
Higuchi, R., et al., "Kinetic PCR analysis: real-time monitoring of DNA amplification reactions", Biotechnology (N Y), vol. 11, pp. 1026-1030 (1993).
Higuchi, R., et al., "Simultaneous amplification and detection of specific DNA sequences", Biotechnology (N Y), vol. 10, pp. 413-417 (1992).
LeDuc, J.W., et al., "Smallpox Research Activities: U.S. Interagency Collaboration, 2001,", Emerg. Infect. Dis., vol. 8, pp. 743-745 (2002).
Leone, G., et al., "Molecular beacon probes combined with amplification by NASBA enable homogeneous, real-time detection of RNA", Nucleic Acids Res., vol. 26, pp. 2150-2155 (1998).
Li, Q., et al., "A New Class of Homogeneous Nucleic Acid Probes Based on Specific Displacement Hybridization," Nucleic Acids Res., vol. 30, article e5 (2002).
Little, M.C., et al., "Strand Displacement Amplification and Homogeneous Real-Time Detection Incorporated in a Second-Generation DNA Probe System, BDProbeTecET", Clin. Chem., vol. 45, pp. 777-784 (1999).
Lizardi, P.M., "Molecular cloning using rolling circle amplification", U.S. Patent Application 20020048761 (2002).
Lizardi, P.M., et al., "Mutation detection and single-molecule counting using isothermal rolling-circle amplification", Nat. Genet., vol. 19, pp. 225-232 (1998).
Merritt, J., et al., "Parallel competition analysis of Saccharomyces cerevisiae strains differing by a single base using polymerase colonies", Nucleic Acids Res., vol. 31, article e84 (2003).
Mitra, R.D., et al., "In situ localized amplification and contact replication of many individual DNA molecules", Nucleic Acids Res., vol. 27, article e34 (1999).
Mitra R.D., et al., "Fluorescent in situ sequencing on polymerase colonies", Anal. Biochem., vol. 320, pp. 55-65 (2003).
Miyoshi, H., et al., "The t(8;21) translocation in acute myeloid leukemia results in production of an AML1-MTG8 fusion transcript", EMBO J., vol. 12, pp. 2715-2721 (1993).
Morrison, T.B., et al., "Quantification of low-copy transcripts by continuous SYBR® Green I monitoring during amplification", BioTechniques, vol. 24, pp. 954-962 (1998).
Muro-Cacho, C., "In situ PCR. Overview of procedures and applications", Frontiers in Bioscience, vol. 2, pp. c15-29 (1997).
Nazarenko, I.A., et al., "Multiplex quantitative PCR using self-quenched primers labeled with a single fluorophore", Nucleic Acids Res., vol. 30, article e37 (2002).
Neidle, S., "DNA Structure and Recognition", Oxford University Press, Oxford, 1994.
Nielsen, P.E. and Egholm, M., Eds., "Peptide Nucleic Acids: Protocols and Applications", Horizon Scientific Press, Wymondham (1999).
Nielsen, P.E., et al., "Sequence-selective recognition of DNA by strand displacement with a thymine-substituted polyamide", Science, vol. 254, pp. 1497-1500 (1991).
Nilsson, M., et al., "Real-time monitoring of rolling-circle amplification using a modified molecular beacon design", Nucleic Acids Res., vol. 30, article e66 (2002).
Notomi, T., et al., "Loop-mediated isothermal amplification of DNA", Nucleic Acids Res., vol. 28, article e63 (2000).
O'Leary, J.J., et al., "The importance of fixation procedures on DNA template and its suitability for solution phase polymerase chain reaction and PCR in-situ hybridization", Histochemical J., vol. 26, pp. 337-346 (1994).
Ortiz, E., et al., "PNA molecular beacons for rapid detection of PCR amplicons", Mol. Cell. Probes, vol. 12, pp. 219-226 (1998).
Pierce, K.E., et al., "Linear-After-The-Exponential (LATE)-PCR: Primer design criteria for high yields of specific singlestranded DNA and improved real-time detection", Proc. Natl. Acad. Sci. USA, vol. 102, pp. 8609-8614 (2005).
Pitner, J.B., et al., "Detection of nucleic acids using G-quartets and I-tetraplexes", U.S. Patent 5,888,739 (1999).
Rhodes, D. et al., "Telomere structure and function" Curr. Opin. Struct. Biol. 5:311-322 (1995).
Samatov, T.R., et al., "Expressible molecular colonies", Nucleic Acids Res., vol. 33, article el45 (2005).
Sambrook, J., et al., "Molecular Cloning", 3rd edn., Cold Spring Harbor Laboratory Press, Cold Spring Harbor, NY (2001).
Sanchez, J.A., et al., "Linear-After-The-Exponential (LATE)-PCR: An advanced method of asymmetric PCR and its uses in quantitative real-time analysis", Proc. Natl. Acad. Sci. USA, vol. 101, pp. 1933-1938 (2004).
Shendure, J., et al., "Accurate multiplex polony sequencing of an evolved bacterial genome", Science, vol. 309, pp. 1728-1732 (2005).
Shirnkus, M., et al., "A chemically cleavable biotinylated nucleotide: Usefulness in the recovery of protein-DNA complexes from avidin affinity columns", Proc. Natl. Acad. Sci. USA, vol. 82, pp. 2593-2597 (1985).
Souazé, F., et al., "Quantitative RT-PCR: Limits and accuracy", BioTechniques, vol. 21, pp. 280-285 (1996).
Stavrianopoulos, J., et al., "Analyte detection by means of energy transfer", U.S. Patent 4,868,103 (1989).
Stewart, C., et al., "Process for performing PCR in mammalian cells", U.S. Patent 5,436,144 (1995).
Stryer, L., et al., "Energy transfer: a spectroscopic ruler", Proc. Natl. Acad. Sci. USA, vol. 58, pp. 719-726 (1967).
Todd, A.V., et al., "DzyNA-PCR: Use of DNAzymes to Detect and Quantify Nucleic Acid Sequences in a Real-Time Fluorescent Format", Clin. Chem., vol. 46, pp. 625-630 (2000).
Tyagi, S., et al., "Extremely sensitive, background-free gene detection using binary probes and Qβ replicase", Proc. Natl. Acad. Sci. USA, vol. 93, pp. 5395-5400 (1996).
Uhlmann, V., et al., "In cell amplification", J. Clin. Pathol.: Mol. Pathol., vol. 51, pp. 119-130 (1998).
van Dongen, J.J.M., et al., "Standardized RT-PCR analysis of fusion gene transcripts from chromosome aberrations in acute leukemia for detection of minimal residual disease. Report of the BIOMED-1 Concerted Action: Investigation of minimal residual disease in acute leukemia", Leukemia, vol. 13, pp. 1901-1928 (1999).
Viehmann, S., et al., "Monitoring of minimal residual disease (MRD) by real-time quantitative reverse transcription PCR (RQ-RT-PCR) in childhood acute myeloid leukemia with AML1/ETO rearrangement", Leukemia, vol. 17, pp. 1130-1136 (2003).
Walker, G.T., et al., "Isothermal in vitro amplification of DNA by a restriction enzyme/DNA polymerase system", Proc. Natl. Acad. Sci. USA, vol. 89, pp. 392-396 (1992).
Willhauck, M., et al., "Internal control for quality assurance of diagnostic RT-PCR", BioTechniques, vol. 25, pp. 656-659 (1998).
Wittwer, C.T., et al., "Continuous Fluorescence Monitoring of Rapid Cycle DNA Amplification", BioTechniques, vol. 22, pp. 130-138 (1997a).
Wittwer, C.T., et al., "The LightCycler™: A Microvolume Multisample Fluorimeter with Rapid Temperature Control", BioTechniques, vol. 22, pp. 176-181 (1997b).
Zhu, J., et al., "Single molecule profiling of alternative pre-mRNA splicing", Science, vol. 301, pp. 836-838 (2003).

A number of examples of the invention have been described.

## Claims

1. A method for detecting the presence in a nucleic acid-containing sample of at least one nucleic acid target sequence, comprising
(a) providing in a sealable reaction chamber an immobilized medium that includes an aqueous liquid phase containing a primer-dependent exponential nucleic acid amplification system for producing at least one amplified product from at least one nucleic acid target sequence and a homogeneous fluorescent reporter system capable of reporting said at least one amplified target, said reporter system including at least one fluorescently labeled primer or hybridization probe whose signaling depends upon incorporation into or hybridization to the amplified product, and that includes a water-insoluble solid matrix entrapping said liquid phase; and wherein nucleic acids of the sample have been distributed in the matrix in a 2-D pattern;
(b) incubating the reaction chamber under conditions providing for amplification of said at least one target sequence to create amplified-product colonies; and
(c) detecting fluorescence in said colonies from the homogeneous fluorescent reporter system without post-amplification processing.

2. The method of claim 1 wherein step (a) comprises soaking a dried gel matrix in an aqueous solution containing the amplification system and the reporter system.

3. The method of claim 1 wherein the immobilized medium is prepared by casting a gel with the amplification system and fluorescent reporter system.

4. The method of any of claims 1 to 3 wherein the homogeneous fluorescent reporter system utilizes energy-transfer, optionally energy transfer FRET.

5. The method of any of claims 1 to 4 wherein the at least one target sequence is RNA, wherein the amplification system includes a reverse transcriptase or a DNA polymerase having reverse transcriptase activity.

6. The method of any of claims 1 to 5 wherein the primer-dependent amplification system is a polymerase chain reaction (PCR) system.

7. The method of any of claims 1 to 6 wherein the reaction chamber is sealed prior to incubation, and detection is performed without opening the reaction chamber.

8. The method of any of claims 1 to 6 further comprising sealing the reaction chamber containing the immobilized medium and nucleic acids of this sample sufficiently to prevent dehydration of the aqueous liquid phase and to prevent escape of amplified product.

9. The method of any of claims 1 to 8 wherein the amplification system includes at least one primer covalently linked to the matrix.

10. The method of any of claims 1 to 8 wherein the sample is cells, in the form of individual cells or tissue fragments, and the process of distributing nucleic acids of the sample in the matrix comprises
(a) embedding the cells in a gel,
(b) lysing the embedded cells,
(c) washing the gel matrix under conditions causing the at least one nucleic acid target sequence to precipitate, with a washing solution comprising a water-miscible organic solvent sufficiently to remove lysing reagents and other substances capable of inhibiting amplification of the at least one target sequence, and
(d) drying the gel matrix to remove the solvent.

11. The method of claim 10, wherein the cells are embedded as a monolayer in the gel.

12. The method of claim 11, wherein the gel is cast onto a layer of a dried second gel that rehydrates to form a merged gel having the cells concentrated beneath its surface.

## Patentansprüche

1. Verfahren zum Nachweisen des Vorliegens wenigstens einer Nukleinsäurezielsequenz in einer nukleinsäurehaltigen Probe, Folgendes umfassend:
(a) Bereitstellen, in einer verschließbaren Reaktionskammer, eines immobilisierten Mediums, das eine wässrige Flüssigphase enthält, welche Folgendes beinhaltet: ein Primer-abhängiges exponentielles Nukleinsäureamplifikationssystem zum Herstellen wenigstens eines amplifizierten Produkts aus wenigstens einer Nukleinsäurezielsequenz und einem homogenen fluoreszierenden Reportersystem, das in der Lage ist, das wenigstens eine amplifizierte Ziel nachzuweisen, wobei das Reportersystem wenigstens einen fluoreszenzmarkierten Primer oder eine Hybridisierungssonde enthält, deren Signalisieren von dem Integrieren in das oder von dem Hybridisieren zum amplifizierten Produkt abhängt, und das eine wasserunlösliche Feststoffmatrix, die die Flüssigphase einschließt, enthält; und wobei die Nukleinsäuren der Probe in einem 2-D-Muster in der Matrix verteilt worden sind;
(b) Inkubieren der Reaktionskammer unter Bedingungen, die ein Amplifizieren der wenigstens einen Zielsequenz gewährleisten, um amplifizierte Produktkolonien zu erzeugen; und
(c) Nachweisen von Fluoreszenz in den Kolonien von dem homogenen fluoreszierenden Reportersystem ohne Verarbeiten nach dem Amplifizieren.

2. Verfahren nach Anspruch 1, wobei Schritt (a) das Einweichen einer Trockengelmatrix in einer wässrigen Lösung, die das Amplifizierungssystem und das Reportersystem beinhaltet, umfasst.

3. Verfahren nach Anspruch 1, wobei das immobilisierte Medium durch Gießen eines Gels mit dem Amplifizierungssystem und dem fluoreszierenden Reportersystem hergestellt wird.

4. Verfahren nach einem der Ansprüche 1 bis 3, wobei das homogene fluoreszierende Reportersystem Energietransfer, optional FRET-Energietransfer nutzt.

5. Verfahren nach einem der Ansprüche 1 bis 4, wobei es sich bei der wenigstens einen Zielsequenz um RNA handelt, wobei das Amplifizierungssystem eine reverse Transkriptase oder eine DNA-Polymerase mit reverser Transkriptaseaktivität enthält.

6. Verfahren nach einem der Ansprüche 1 bis 5, wobei das Primer-abhängige Amplifizierungssystem ein Polymerase-Kettenreaktions(PCR)-System ist.

7. Verfahren nach einem der Ansprüche 1 bis 6, wobei die Reaktionskammer vor dem Inkubieren verschlossen wird und das Nachweisen ohne Öffnen der Reaktionskammer durchgeführt wird.

8. Verfahren nach einem der Ansprüche 1 bis 6, ferner umfassend das Verschließen der Reaktionskammer, die das immobilisierte Medium und Nukleinsäuren dieser Probe ausreichend enthält, um das Dehydrieren der wässrigen Flüssigphase zu verhindern und das Austreten von amplifiziertem Produkt zu verhindern.

9. Verfahren nach einem der Ansprüche 1 bis 8, wobei das Amplifizierungssystem wenigstens einen Primer, der kovalent mit der Matrix verknüpft ist, enthält.

10. Verfahren nach einem der Ansprüche 1 bis 8, wobei es sich bei der Probe um Zellen in der Form von individuellen Zellen oder Gewebefragmenten handelt und der Vorgang des Verteilens von Nukleinsäuren der Probe in der Matrix Folgendes umfasst:
(a) Einlagern der Zellen in einem Gel,
(b) Lysieren der eingelagerten Zellen,
(c) Waschen der Gelmatrix unter Bedingungen, die bewirken, dass die wenigstens eine Nukleinsäurezielsequenz ausfällt, mit einer Waschlösung, umfassend ein wassermischbares organisches Lösungsmitttel, ausreichend, um Lysereagenzien und andere Substanzen, die in der Lage sind, das Amplifizieren der wenigstens einen Zielsequenz zu hemmen, zu entfernen, und
(d) Trocknen der Gelmatrix, um das Lösungsmittel zu entfernen.

11. Verfahren nach Anspruch 10, wobei die Zellen als eine Monoschicht in dem Gel eingelagert werden.

12. Verfahren nach Anspruch 11, wobei das Gel auf eine Schicht aus einem getrockneten zweiten Gel gegossen wird, das rehydriert, um ein vermischtes Gel auszubilden, bei dem die Zellen unter seiner Oberfläche konzentriert sind.

## Revendications

1. Procédé de détection de la présence d'au moins une séquence d'acide nucléique cible dans un échantillon contenant un acide nucléique, qui comprend
(a) la fourniture, dans une cuve de réaction à fermeture hermétique, d'un milieu immobilisé qui comprend une phase liquide aqueuse contenant un système d'amplification exponentielle des acides nucléiques nécessitant une amorce pour obtenir au moins un produit amplifié à partir d'au moins une séquence d'acide nucléique cible et un système rapporteur fluorescent homogène capable de signaler ladite au moins une cible amplifiée, ledit système rapporteur incluant au moins une amorce marquée par fluorescence ou une sonde d'hybridation dont le signal dépend de l'incorporation dans ou de l'hybridation au produit amplifié, et qui comprend une matrice solide insoluble dans l'eau piégeant ladite phase liquide et dans laquelle les acides nucléiques de l'échantillon ont été distribués selon un modèle 2-D ;
(b) l'incubation de la cuve de réaction dans des conditions appropriées pour l'amplification de ladite au moins une séquence cible pour créer des colonies de produits amplifiés ; et
(c) la détection de la fluorescence dans lesdites colonies au moyen du système rapporteur fluorescent homogène sans traitement de post-amplification.

2. Procédé selon la revendication 1, où l'étape (a) comprend l'imbibition d'une matrice de gel sec dans une solution aqueuse contenant le système d'amplification et le système rapporteur.

3. Procédé selon la revendication 1, où le milieu immobilisé est préparé en moulant un gel avec le système d'amplification et le système rapporteur fluorescent.

4. Procédé selon l'une quelconque des revendications 1 à 3, où le système rapporteur fluorescent homogène utilise un transfert d'énergie, en option un transfert d'énergie par résonance de type Förster (FRET).

5. Procédé selon l'une quelconque des revendications 1 à 4, où la au moins une séquence cible est un ARN et où le système d'amplification inclut une transcriptase inverse ou une ADN-polymérase à activité transcriptase inverse.

6. Procédé selon l'une quelconque des revendications 1 à 5, où le système d'amplification nécessitant une amorce est un système de réaction en chaîne par polymérase (PCR).

7. Procédé selon l'une quelconque des revendications 1 à 6, où la cuve de réaction est hermétiquement fermée avant l'incubation et où la détection est effectuée sans ouvrir la cuve de réaction.

8. Procédé selon l'une quelconque des revendications 1 à 6, qui comprend en outre la fermeture hermétique de la cuve de réaction contenant le milieu immobilisé et des acides nucléiques de cet échantillon d'une manière suffisante pour empêcher la déshydratation de la phase liquide aqueuse et la fuite du produit amplifié.

9. Procédé selon l'une quelconque des revendications 1 à 8, où le système d'amplification inclut au moins une amorce liée de manière covalente à la matrice.

10. Procédé selon l'une quelconque des revendications 1 à 8, où l'échantillon correspond à des cellules sous la forme de cellules individuelles ou de fragments d'un tissu, et où le procédé de distribution des acides nucléiques de l'échantillon dans la matrice comprend
(a) l'inclusion des cellules dans un gel,
(b) la lyse des cellules incluses,
(c) le lavage de la matrice de gel, dans des conditions qui causent la précipitation de la au moins une séquence d'acide nucléique cible, avec une solution de lavage comprenant un solvant organique miscible à l'eau d'une manière suffisante pour éliminer les réactifs de lyse et autres substances capables d'inhiber l'amplification de la au moins une séquence cible, et
(d) le séchage de la matrice de gel pour éliminer le solvant.

11. Procédé selon la revendication 10, où les cellules sont incluses dans le gel sous la forme d'une monocouche.

12. Procédé selon la revendication 11, où le gel est moulé sur une couche d'un deuxième gel sec qui se réhydrate pour former un gel fusionné dans lequel les cellules sont concentrées sous sa surface.
